# DEMANDE DE BREVET EUROPEEN

(11) **EP 2 072 084 A2**
(43) Date de publication de la demande: **24.06.2009**
(21) Numéro de dépôt: 08164229.0
(22) Date de dépôt: 12.09.2008
(51) Int. Cl.: A61Q 5/12, A61Q 5/02, A61K 8/73, A61K 8/81, A61K 8/898

(54) **Compositions cosmétiques contenant un copolymère cationique, une silicone aminée et un polymère cationique et leurs utilisations**

(30) Priorité: 14.09.2007 FR 0757579
(71) Demandeur: L'Oréal, 75008 Paris (FR)
(72) Inventeur: Mahe, Véronique, 78740 VAUX S/SEINE (FR); Biganska, Olga, 92340 Bourg La Reine (FR)
(74) Mandataire: Le Blainvaux Bellegarde, Françoise

(57) **Abrégé**

L'invention concerne de nouvelles compositions cosmétiques comprenant dans un milieu cosmétiquement acceptable :
(i)- un ou plusieurs polymères cationiques qui sont produits par polymérisation d'un mélange de monomères comprenant :
a) un ou plusieurs monomères vinyliques substitués par un ou plusieurs groupes amino,
b) un ou plusieurs monomères vinyliques non ioniques hydrophobes, et
c) un ou plusieurs monomères vinyliques associatifs,
e) un ou plusieurs monomères vinyliques non ioniques hydroxylés,

(ii)- une ou plusieurs silicones aminées, et
(iii)- un ou plusieurs polymères cationiques différents des polymères cationiques (i) et des silicones aminées (ii) et présentant une densité de charge cationique supérieure ou égale à 4 meq/g.

Ces compositions sont utilisées notamment pour le lavage et/ou le conditionnement des matières kératiniques telles que les cheveux ou la peau.

## Description

La présente invention concerne de nouvelles compositions cosmétiques comprenant dans un milieu cosmétiquement acceptable un ou plusieurs copolymères cationiques particuliers, une ou plusieurs silicones aminées et un ou plusieurs polymères cationiques particuliers.

Il est bien connu que des cheveux qui ont été sensibilisés (i.e. abîmés et/ou fragilisés) à des degrés divers sous l'action d'agents atmosphériques ou sous l'action de traitements mécaniques ou chimiques, tels que des colorations, des décolorations et/ou des permanentes, sont souvent difficiles à démêler et à coiffer, et manquent de douceur.

On a déjà préconisé dans les compositions pour le lavage ou le soin des matières kératiniques telles que les cheveux l'utilisation d'agents conditionneurs, notamment des polymères cationiques ou amphotères ou des silicones, pour faciliter le démêlage des cheveux et pour leur communiquer douceur et souplesse. Cependant, les avantages cosmétiques mentionnés ci-avant s'accompagnent malheureusement également, sur cheveux séchés, de certains effets cosmétiques jugés indésirables, à savoir un alourdissement de la coiffure (manque de légèreté du cheveu), un manque de lissage (cheveu non homogène de la racine à la pointe).
En outre, l'usage des polymères cationiques ou amphotères dans ce but présente divers inconvénients. En raison de leur forte affinité pour les cheveux, certains de ces polymères se déposent de façon importante lors d'utilisations répétées, et conduisent à des effets indésirables tel qu'un toucher désagréable, chargé, un raidissement des cheveux, et une adhésion interfibres affectant le coiffage. Ces inconvénients sont accentués dans le cas de cheveux fins, qui manquent de nervosité et de volume.

Les silicones aminées sont généralement utilisées dans les compositions de shampooings en tant qu'agents conditionneurs pour améliorer la douceur, le toucher et le démêlage des cheveux. Cependant, on a constaté que ces silicones conduisaient à la formation d'une couche inesthétique à la surface du shampooing nuisible aux performances du shampooing. Pour éviter l'apparition de ce phénomène, des agents de stabilisation tels que les polymères acryliques réticulés du type Carbopol 980 sont fréquemment utilisés. Néanmoins, ces agents de stabilisation présentent l'inconvénient de diminuer les performances cosmétiques des shampooings, notamment en rendant les cheveux plus chargés et plus rêches.

Les polymères de dialkyl diallyl ammonium sont souvent utilisés dans des compositions de shampooings pour améliorer les propriétés cosmétiques des cheveux sensibilisés, par exemple pour les rendre moins rêches et/ou améliorer leur aptitude au démêlage.
Cependant, on a constaté que ces polymères de dialkyl diallyl ammonium présentaient souvent l'inconvénient de diminuer les performances cosmétiques des cheveux naturels, notamment en les alourdissant.
Il était donc nécessaire de mettre au point une composition cosmétique détergente, en particulier un shampooing, qui permette d'obtenir des performances cosmétiques acceptables sur les matières kératiniques, à savoir notamment les cheveux et le cuir chevelu, et plus particulièrement sur les cheveux naturels.

En résumé, il s'avère que les compositions cosmétiques actuelles contenant des polymères cationiques, ne donnent pas encore complètement satisfaction.

On connaît dans l'état de la technique des compositions cosmétiques en particulier détergentes contenant un copolymère cationique comme agent de stabilisation ou de suspension d'ingrédients insolubles dans l'eau comme des silicones ou des corps gras. De telles compositions ont été décrites notamment dans la demande de brevet WO2005/092276. Les qualités de mousse et les propriétés cosmétiques obtenues avec ces compositions ne sont pas encore suffisamment satisfaisantes, de même que les propriétés cosmétiques.
La revue Cosmetic Science Technology de 2006 page 247 divulgue une composition comprenant un copolymère acrylique tel que le Carbopol Aqua CC, des silicones aminées et un polymère cationique présentant une densité de charge cationique inférieure à 4 meq/g , le polyquaternium-10.
FR2798849 décrit l'association d'un terpolymère acrylique tel que le Structure Plus et d'un homopolymère de diallyl dialkylammonium.
FR2798849 décrit l'association d'un terpolymère acrylique tel que le Structure Plus et d'une silicone aminée.

La demanderesse a maintenant découvert que l'association d'un copolymère cationique particulier, d'un deuxième polymère cationique particulier et d'une silicone particulière permet de remédier à ces inconvénients.

En effet, il a été constaté que l'utilisation dudit copolymère cationique dans les compositions de la présente invention permettait d'obtenir sur les matières kératiniques, notamment les cheveux de très bonnes propriétés cosmétiques particulièrement au niveau de la légèreté, de la douceur, du lissage au toucher, au niveau de la souplesse et de la malléabilité sur cheveux séchés. Il a aussi été constaté que les compositions de l'invention permettaient d'obtenir des cheveux séchés présentant au regard un aspect général plus lisse.

Par ailleurs, les compositions selon l'invention sont stables et présentent un aspect visuel esthétique. Les propriétés d'usage (aspect, consistance, abondance de la mousse, élimination de la mousse) sont très satisfaisantes.

Les compositions de l'invention appliquées sur la peau notamment sous forme de bain moussant ou de gel douche, apportent une amélioration de la douceur de la peau.

Ainsi, selon la présente invention, il est maintenant proposé de nouvelles compositions cosmétiques, comprenant, dans un milieu cosmétiquement acceptable :
(i)- un ou plusieurs polymères cationiques qui sont produits par polymérisation d'un mélange de monomères comprenant :
   a) un ou plusieurs monomères vinyliques substitués par un ou plusieurs groupes amino,
   b) un ou plusieurs monomères vinyliques non ioniques hydrophobes,
      choisis parmi ceux de formules (I) et (II) :

      CH₂=C(X)Z, (I)

      CH₂=CH-OC(O)R; (II)

      dans lesquelles formules (I) et (II) :
      X représente H ou un groupe méthyle ;
      Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
      x représente un nombre entier allant de 1 à 6 ;
         chaque R représente indépendamment un groupe alkyle en C₁-C₃₀ ;
         chaque R¹ représente indépendamment un groupe alkyle en C₁-C₃₀ , un groupe alkyle en C₂-C₃₀ hydroxylé, ou un groupe alkyle en C₁-C₃₀ halogéné et
   c) un ou plusieurs monomères vinyliques associatifs,
   e) un ou plusieurs monomères vinyliques non ioniques hydroxylés,
(ii)- une ou plusieurs silicones aminées, et
(iii)- un ou plusieurs polymères cationiques différents des polymères cationiques (i) et des silicones aminées (ii) et présentant une densité de charge cationique supérieure ou égale à 4 meq/g.

L'invention a également pour objet l'utilisation d'une composition telle que définie ci-dessus pour apporter une ou plusieurs propriétés choisies parmi la brillance, la légèreté des cheveux, la douceur, le lissage au toucher, et/ou la souplesse des cheveux.

Un autre objet de l'invention concerne un procédé de traitement des matières kératiniques, telles que les cheveux, caractérisé en ce qu'il consiste à appliquer sur lesdites matières des compositions cosmétiques selon l'invention.

Selon la présente invention, par matières kératiniques, on comprend les cheveux, les cils, les sourcils, la peau, les ongles, les muqueuses ou le cuir chevelu et plus particulièrement les cheveux.
Par monomère hydrophobe, on entend au sens de la présente invention, un monomère qui présente une solubilité dans l'eau inférieure à 10g pour 100 mL d'eau à une température de 20°C.

Un autre objet de l'invention concerne l'utilisation d'un copolymère cationique (i) tel que décrit ci-dessus dans, ou pour la fabrication d'une composition cosmétique comprenant un ou plusieurs polymères cationiques (iii) différents du ou des polymères cationiques (i) et présentant une densité de charge cationique supérieure ou égale à 4 meq/g et une ou plusieurs silicones aminées.

Les différents objets de l'invention vont maintenant être détaillés. L'ensemble des significations et définitions des composés utilisés dans la présente invention donné ci-dessous sont valables pour l'ensemble des objets de l'invention.

L'une des caractéristiques essentielles de l'invention est la présence d'un polymère cationique qui est obtenu par polymérisation d'un mélange de monomères comprenant a) un ou plusieurs monomères vinyliques substitués par un ou plusieurs groupes amines, b) un ou plusieurs monomères vinyliques non ioniques hydrophobes, c) un ou plusieurs monomères vinyliques associatifs et e) un ou plusieurs monomères vinyliques non ioniques hydroxylés. De préférence, les monomères constituant le copolymère cationique comprennent en outre un ou plusieurs monomères tensioactifs vinyliques semi-hydrophobes e). Les monomères a) à e) sont différents les uns des autres.

De préférence, le polymère cationique (i) est un polymère épaississant.

Au sens de la présente invention, on entend par polymère épaississant, un polymère qui, introduit à 1 % en poids dans une solution aqueuse ou hydroalcoolique à 30 % en poids d'éthanol, et à pH 7, permet d'atteindre une viscosité d'au moins 100 cps à 25 °C, et à un taux de cisaillement de 1 s⁻¹. Cette viscosité peut être mesurée à l'aide d'un viscosimètre à géométrie cône-plan, par exemple, un rhéomètre Haake RS 600. De préférence, ces polymères permettent d'augmenter la viscosité des compositions dans lesquelles ils se trouvent, d'au moins 50 cps à 25 °C et à 1 s⁻¹.

Le ou les polymères cationiques (i) utilisés dans la composition selon l'invention, et leur procédé de fabrication, sont notamment décrits dans la demande internationale WO 2004/024779.

Par monomère vinylique, on entend au sens de la présente invention un monomère comprenant un ou plusieurs groupes R₀CH=C(R₀)- , ou chaque R₀ est indépendamment H, un alkyle en C₁-C₃₀, -C(O)OH ou C(O)OR₀', -OC(O)OR₀', -C(O)NHR₀', C(O)NRo' R₀" ,R₀' et Ro", identiques ou différents, étant un groupe alkyle en C₁-C₃₀.
Ainsi, par exemple, au sens de la présente invention, les (méth)acrylates et les (méth)acrylamides sont des monomères vinyliques.
Comme expliqué précédemment, le mélange de monomères permettant la préparation du polymère cationique (i) utilisé dans la composition selon l'invention comprend un ou plusieurs monomères vinyliques substitués par un
ou plusieurs groupes amino.
Les monomères vinyliques substitués par un ou plusieurs groupes amino utilisables pour la préparation du polymère cationique utilisé selon l'invention sont des monomères à insaturation éthylénique, basiques et polymérisables. Les groupes amines peuvent dériver de groupes alkyles mono, di- ou polyaminés, ou bien de groupes hétéroaromatiques comprenant un atome d'azote. Les groupes amines peuvent être des amines primaires, secondaires
ou tertiaires. Ces monomères peuvent être utilisés sous la forme d'amine ou sous la forme de sel.
De préférence, le ou les monomères vinyliques substitués par un ou plusieurs groupes amines sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides à groupement(s) hétérocyclique(s) contenant un atome d'azote,
- les (méth)acrylates à groupement(s) hétérocyclique(s) contenant un atome d'azote,
- les hétérocycles azotés à groupement(s) vinyle(s),
   et leurs mélanges.
   A titre de monomères vinyliques substitués par un ou plusieurs groupes amino préférés, on peut citer :

- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.
   Lorsque les monomères sont sous forme de sels, il peut s'agir de sels minéraux, tels que les sels hydrochlorure, sulfate et phosphate ; ou bien de sels d'acides organiques, tels que les sels acétate, maléate et fumarate.
   Des monomères vinyliques substitués par un ou plusieurs groupe(s) amino particulièrement préférés sont :
- le (méth)acrylate de 3-(N,N-diméthylamino)propyle,
- le N'-(3-N,N-diméthylamino)propyl(méth)acrylamide,
- le (méth)acrylate de 2-(N,N-diméthylamino)éthyle,
- le (méth)acrylate de 2-(N,N-diéthylamino)éthyle,
- le (méth)acrylate de 2-(tert-butylamino)éthyle,
- le 2-(N,N-diméthylamino)propyl(méth)acrylamide, et
- l'acrylate de 2-(N,N-diméthylamino)néopentyle.
   Le ou les monomères vinyliques substitués par un ou plusieurs groupes amino représentent généralement de 10 à 70 % en poids, de préférence de 20 à 60 % en poids, mieux de 30 à 40 % en poids, par rapport au poids total du mélange de monomères.

Comme expliqué précédemment, le mélange de monomères permettant la préparation du polymère cationique utilisé (i) selon l'invention comprend également un ou plusieurs monomères vinyliques non ioniques hydrophobes b).

Le ou les monomères vinyliques non ioniques hydrophobes utilisables pour la préparation du polymère cationique utilisé selon l'invention sont généralement choisis parmi les composés de formules (I) et (II) :

CH₂=C(X)Z, (I)

CH₂=CH-OC(O)R; (II)

où, dans chacune des formules (I) et (II) ;
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀, un groupe alkyle en C₂-C₃₀ hydroxylé, ou un groupe alkyle en C₁-C₃₀ halogéné.
On peut citer en particulier les (méth)acrylates d'alkyle en C₁-C₃₀ ; les (alkyl en C₁-C₃₀)(méth)acrylamides ; le styrène, les styrènes substitués et en particulier le vinyltoluène (ou 2-méthylstyrène), le butylstyrène, l'isopropylstyrène, le para-chlorostyrène ; les esters de vinyle et en particulier l'acétate de vinyle, le butyrate de vinyle, le caprolate de vinyle, le pivalate de vinyle et le néodécanoate de vinyle ; les nitriles insaturés et en particulier le (méth)acrylonitrile et l'acrylonitrile ; et les silanes insaturés et en particulier le triméthylvinylsilane, le diméthyléthylvinylsilane, l'allyldiméthylphénylsilane, l'allyltriméthylsilane, le 3-acrylamidopropyltriméthylsilane, le méthacrylate de 3-triméthylsilylpropyle.
De préférence, le ou les monomères vinyliques non ioniques hydrophobes sont choisis parmi les acrylates d'alkyle en C₁-C₃₀, les méthacrylates d'alkyle en C₁-C₃₀, et leurs mélanges, tels que l'acrylate d'éthyle, le méthacrylate de méthyle, le méthacrylate de 3,3,5-triméthylcyclohexyle.
Le ou les monomères vinyliques non ioniques hydrophobes représentent généralement de 20 à 80% en poids, de préférence de 20 à 70% en poids, mieux de 50 à 65 % en poids, par rapport au poids total du mélange de monomères.

Le ou les monomères vinyliques associatifs utilisables pour la préparation du polymère cationique (i) utilisé selon l'invention sont généralement choisis parmi des composés ayant une extrémité (i)' à insaturation(s) éthylénique(s) pour la polymérisation par addition avec d'autres monomères du système ; une portion centrale (ii)' polyoxyalkylène pour conférer des propriétés hydrophiles sélectives aux polymères, et une extrémité (iii)' hydrophobe pour conférer des propriétés hydrophobes sélectives aux polymères.
L'extrémité (i)' à insaturation(s) éthylénique(s) du ou des monomères vinyliques associatifs est de préférence dérivée d'un acide ou d'un anhydride mono ou di-carboxylique à insaturation(s) α, β-éthylénique(s), de préférence un acide ou un anhydride mono ou di-carboxylique en C₃ ou C₄. De façon alternative, l'extrémité (i)' du monomère associatif peut être dérivée d'un allyl éther ou d'un vinyl éther ; d'un monomère non ionique uréthane substitué par un groupe vinyle, tel que divulgué dans le brevet US redélivré n°33,156 ou dans le brevet US 5,294,692 ; ou d'un produit de réaction urée substituée par un groupe vinyle, tel que divulgué dans le brevet US 5,011,978.
La portion centrale (ii)' du ou des monomères vinyliques associatifs est de préférence un segment polyoxyalkylène comprenant 5 à 250, de préférence encore 10 à 120, et mieux 15 à 60 unités oxydes d'alkylène en C₂-C₇. Des portions centrales (ii)' préférées sont les segments polyoxyéthylène, polyoxypropylène, et polyoxybutylène comprenant 5 à 150, de préférence 10 à 100, et mieux 15 à 60 unités oxydes d'éthylène, de propylène ou de butylène, et des séquences aléatoires ou non aléatoires d'unités oxydes d'éthylène, oxydes de propylène ou oxydes de butylène. De préférence, les portions centrales sont des segments polyoxyéthylène.
L'extrémité (iii)' hydrophobe du ou des monomères associatifs est de préférence un fragment hydrocarboné appartenant à l'une des classes hydrocarbonées suivantes : un alkyle linéaire en C₈-C₄₀, un alkyle en C₂-C₄₀ substitué par un groupe aryle, un phényle substitué par un groupe alkyle en C₂-C₄₀, un alkyle ramifié en C₈-C₄₀, un groupe alicyclique en C₈-C₄₀, et un ester complexe en C₈-C₈₀.
Par ester complexe, on entend au sens de la présente invention tout ester différent d'un ester simple.
Par ester simple, on entend au sens de la présente invention tout ester d'alcool aliphatique saturé en C₁-C₃₀, linéaire ou ramifié, et non substitué.

Des exemples d'extrémités (iii)' hydrophobes du ou des monomères associatifs sont des groupes alkyles linéaires ou ramifiés ayant de 8 à 40 atomes de carbone, tels que les groupes capryle (C₈), isooctyle (C₈ ramifié), décyle (C₁₀), lauryle (C₁₂), myristyle (C₁₄), cétyle (C₁₆), cétéaryle (C₁₆-C₁₈), stéaryle (C₁₈), isostéaryle (C₁₈ ramifié), arachidyle (C₂₀), béhényle (C₂₂), lignocéryle (C₂₄), cérotyle (C₂₆), montanyle (C₂₈), mélyssile (C₃₀) et laccéryle (C₃₂).
Des exemples de groupes alkyles linéaires ou ramifiés ayant 8 à 40 atomes de carbone et dérivés d'une source naturelle sont notamment les groupes alkyles dérivés de l'huile d'arachide hydrogénée, d'huile de soja et d'huile de canola (à prédominance C₁₈), l'huile de suif hydrogénée en C₁₆-C₁₈ ; et les terpénols hydrogénés en C₁₀-C₃₀, tels que le géraniol hydrogéné (en C₁₀ ramifié), le farnesol hydrogéné (C₁₅ ramifié), le phytol hydrogéné (C₂₀ ramifié).
Des exemples de phényles substitués par un alkyle en C₂-C₄₀ sont l'octylphényle, le nonylphényle, le décylphényle, le dodécylphényle, l'hexadécylphényle, l'octadécylphényle, l'isooctylphényle et le sec-butylphényle.
Des groupes alicycliques en C₈-C₄₀ peuvent être par exemple les groupes dérivés des stérols d'origine animale, tels que le cholestérol, le lanostérol, le 7-déhydrocholestérol ; ou bien les dérivés d'origine végétales, tels que le phytostérol, le stigmastérol, le campestérol ; ou bien les dérivés issus de microorganismes, tels que l'ergostérol, le mycrostérol. D'autres alicycliques en C₈-C₄₀ utilisables dans la présente invention sont par exemple le cyclooctyle, le cyclododécyle, l'adamantyle, le décahydronaphthyle, et les groupes dérivés de composés alicycliques en C₈-C₄₀ naturels tels que le pinène, le rétinol hydrogéné, le camphre et l'alcool d'isobornyle.
Les groupes alkyles en C₂-C₄₀ substitués par un groupe aryle peuvent être par exemple le 2-phényléthyle, le 2,4-diphénybutyle, le 2,4,6-triphénylhexyle, le 4-phénylbutyle, le 2-méthyl-2-phényléthyle, le 2,4,6-tri(1'phényléthyl)phényle.
A titre d'esters complexes en C₈-C₄₀ utilisables comme extrémité (iii), on peut citer l'huile de ricin hydrogénée (principalement le triglycéride de l'acide 12-hydroxystéarique ; les 1,2-diacyl glycérols tels que le 1,2-distéaryl glycérol, le 1,2-dipalmityl glycérol, le 1,2-dimyristyl glycérol ; les di-, tri- ou poly-esters de sucres tel que le 3,4,6-tristéaryl glucose, le 2,3-dilauryle fructose ; et les esters de sorbitane tels que ceux divulgués dans le brevet US 4,600,761.
Les monomères vinyliques associatifs utilisables selon l'invention peuvent être préparés par toute méthode connue dans l'art antérieur. On peut se référer par exemple aux brevets US 4,421,902, US 4,384,096, US 4,514,552, US 4,600,761, US 4,616,074, US 5,294,692, US 5,292,843 ; US 5,770,760 et US 5,412,142.
De préférence, le ou les monomères vinyliques associatifs c) utilisables selon l'invention sont choisis parmi les composés de formule (III) : 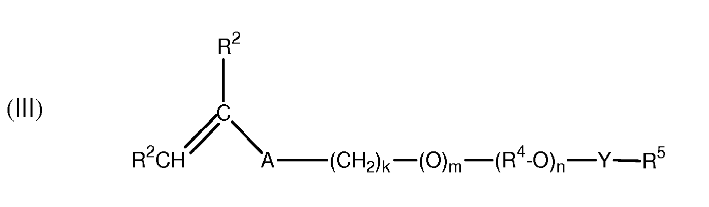 Dans laquelle
chaque R² est indépendamment H, un groupe méthyle, un groupe -C(O)OH, ou un groupe -C(O)OR³ ;
R³ est un alkyle en C₁-C₃₀ ;
A est un groupe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z} -NHC(O)NH-, ou -CH₂CH₂-NHC(O)-;
Ar est un groupe aryle divalent ;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs, avec des unités oxyalkylène en C₂-C₄,
R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou -C(O)NHC(O)-;
R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogèno.
De préférence, le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.
Plus particulièrement, le ou les monomères vinyliques associatifs sont choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, où la portion polyéthoxylée du monomère comprend de 10 à 80, de préférence de 15 à 60 et mieux de 20 à 40 unités oxydes d'éthylène.
De préférence le ou les monomères associatifs vinyliques représentent de 0,001 à 25 % en poids, de préférence de 0,01 à 15 % en poids et mieux de 0,1 à 10 % en poids du mélange de monomères.

Le ou les monomères tensioactifs vinyliques semi-hydrophobes, éventuellement présents dans le mélange de monomères peuvent modérer les propriétés associatives des polymères associatifs cationiques qui les contiennent, produisant ainsi des gels aqueux ayant une très bonne texture et de très bonnes propriétés rhéologiques.
Par monomère tensioactif vinylique semi-hydrophobe, on entend au sens de la présente invention un monomère qui a une structure similaire à un monomère associatif, mais a une extrémité substantiellement non hydrophobe et ainsi ne confère pas de propriété associative aux polymères.
La propriété d'associativité d'un polymère est liée à la propriété dans un milieu donné, des molécules dudit polymère de s'associer entre elles, ou de s'associer à des molécules d'un co-agent, en général tensioactif, ce qui se traduit dans un certain domaine de concentration par un accroissement de la viscosité du milieu.
Le ou les monomères tensioactifs vinyliques semi-hydrophobes sont généralement des composés ayant deux parties :
(i) un groupe terminal insaturé pour permettre la polymérisation par addition avec les autres monomères du mélange de réaction, et
(ii) un groupe polyoxyalkylène pour atténuer les associations entre les groupes hydrophobes du polymère ou les groupes hydrophobes des autres matériaux éventuellement présents dans la composition comprenant le polymère.

L'extrémité fournissant l'insaturation vinylique ou éthylénique pour la polymérisation par addition est de préférence dérivée d'un acide ou d'un anhydride mono ou di-carboxylique à insaturation α, β-éthylénique, de préférence un acide mono ou di-carboxylique en C₃-C₄, ou un anhydride de cet acide. De façon alternative, l'extrémité A peut dériver d'un éther allylique, d'un éther vinylique ou d'un uréthane insaturé non ionique.
L'extrémité A insaturée polymérisable peut aussi dériver d'un acide gras insaturé en C₈-C₃₀ comprenant un ou plusieurs groupe fonctionnel carboxy libre. Ce groupe en C₈-C₃₀ fait partie de l'extrémité insaturée A et est différent des groupes hydrophobes pendant des monomères associatifs, qui sont séparés de l'extrémité insaturée du monomère associatif par un groupe espaceur hydrophile.
La portion B polyoxyalkylène comprend un segment polyoxyalkylène à chaîne longue, qui est essentiellement similaire à la portion hydrophile des monomères associatifs. Des portions polyoxyalkylène B préférées incluent les unités en C₂-C₄ polyoxyéthylène, polyoxypropylène, et polyoxybutylène comprenant de 5 à 250, de préférence de 10 à 100 unités oxyalkylène. Lorsque le monomère tensioactif vinylique semi-hydrophobe comprend plus d'un type d'unité d'oxyalkylène, ces unités peuvent être disposés en séquence aléatoire, non aléatoire, ou à bloc.
De préférence, le ou les monomères tensioactifs vinyliques semi-hydrophobes sont choisis parmi les composés de formules (IV) ou (V) :

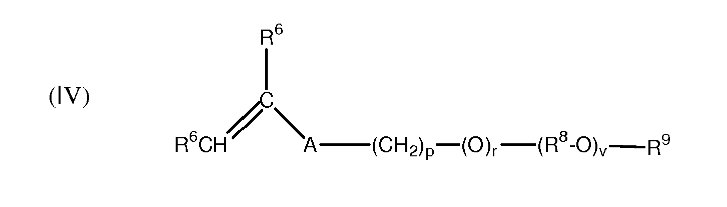

D-A-(CH₂)ₚ-(O)ᵣ-(R⁸-O)ᵥ-R⁹ (V)

où, dans chaque formule (IV) ou (V),
chaque R⁶ représente indépendamment H, un alkyle en C₁-C₃₀, - C(O)OH, ou C(O)OR⁷ ;
R⁷ est un alkyle en C₁-C₃₀ ;
A est un groupe -CH₂C(O)O-, -C(O)O-, -O-, -CH₂O, -NHC(O)NH-, -C(O)NH-, -Ar-(CE₂)_{z}-NHC(O)O-, -Ar-(CE₂)_{z} -NHC(O)NH-, ou -CH₂CH₂NHC(O)-;
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄ ;
D est un alkyle insaturé en C₈-C₃₀ ou un alkyle insaturé en C₈-C₃₀ substitué par un groupe carboxy.
De manière particulièrement préférée, le mélange de monomères comprend un monomère tensioactif vinylique semi-hydrophobe ayant l'une des formules suivantes :

CH₂=CH-O(CH₂)ₐO(C₃H₆O)_{b}(C₂H₄O)_{c}H ou

CH₂=CHCH₂O(C₃H₆O)_{d}(C₂H₄O)ₑH;

où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.
Des monomères tensioactifs vinyliques semi-hydrophobes préférés sont par exemple les émulsifiants polymérisables commercialisés sous les références EMULSOGEN^{®} R109, R208, R307, RAL109, RAL208 et RAL307 par la société CLARIANT ; BX-AA-E5P5 commercialisé par la société BIMAX ; et le MAXEMUL^{®} 5010 et 5011 commercialisé par la société UNIQEMA. Les monomères particulièrement préférés sont l'EMULSOGEN^{®} R208, R307 et RAL 307.

Selon les fabricants :
l'EMULSOGEN^{®} R109 est un éther vinylique 1,4-butanediol éthoxylé/propoxylé aléatoire ayant la formule empirique :

   CH₂=CH-O(CH₂)₄O(C₃H₆O)₄(C₂H₄O)₁₀H;
l'EMULSOGEN^{®} R208 qui est un éther vinylique 1,4-butanediol éthoxylé/propoxylé aléatoire ayant la formule empirique :

   CH₂=CH-O(CH₂)₄O(C₃H₆O)₄(C₂H₄O)₂₀H;
l'EMULSOGEN^{®} R307 qui est un éther vinylique 1,4-butanediol éthoxylé/propoxylé aléatoire ayant la formule empirique :

   CH₂=CH-O(CH₂)₄O(C₃H₆O)₄(C₂H₄O)₃₀H;
l'EMULSOGEN^{®} RAL 109 qui est un éther allylique éthoxylé/propoxylé aléatoire ayant la formule empirique :

   CH₂=CHCH₂-O(C₃H₆O)₄(C₂H₄O) ₁₀H;
l'EMULSOGEN^{®} RAL 208 qui est un éther allylique éthoxylé/propoxylé aléatoire ayant la formule empirique :

   CH₂=CHCH₂-O(C₃H₆O)₄(C₂H₄O) ₂₀H;
l'EMULSOGEN^{®} RAL 307 qui est un éther allylique éthoxylé/propoxylé aléatoire ayant la formule empirique :

   CH₂=CHCH₂-O(C₃H₆O)₄(C₂H₄O) ₃₀H;
le MAXEMUL^{®} 5010 qui est un alcényle en C₁₂-C₁₅ carboxylé hydrophobe, éthoxylé avec 24 unités d'oxyde éthylène,
le MAXEMUL^{®} 5011 qui est un alcényle en C₁₂-C₁₅ carboxylé hydrophobe, éthoxylé avec 34 unités d'oxyde éthylène ;
et le BX-AA-E5P5 qui est un éther allylique éthoxylé/propoxylé aléatoire ayant la formule empirique :

   CH₂=CHCH₂-O(C₃H₆O)₅(C₂H₄O) ₅H.
La quantité du ou des monomères tensioactifs vinyliques semi-hydrophobes utilisés dans la préparation des polymères cationiques (i) utilisés dans la composition selon l'invention peut varier largement et dépend, en autre, des propriétés rhéologiques finales désirées pour le polymère.
Lorsqu'ils sont présents, les monomères tensioactifs vinyliques semi-hydrophobes représentent de 0,01 à 25 % en poids et de préférence de 0,1 à 10% en poids par rapport au poids total du mélange de monomères.

Le ou les polymères cationiques (i) utilisés dans la composition selon l'invention sont préparés à partir d'un mélange de monomères pouvant comprendre un ou plusieurs monomères vinyliques non ioniques hydroxylés.

Ces monomères sont des monomères à insaturation(s) éthylénique(s) comprenant un ou plusieurs substituants hydroxyle.
A titre de monomères vinyliques non ioniques hydroxylés, on peut citer les (méth)acrylates d'alkyle en C₁-C₆ hydroxylés, de préférence les (méth)acrylates d'alkyle en C₁-C₄ hydroxylés, tel que le 2-hydroxyéthylméthacrylate (HEMA), le 2-hydroxyéthyl acrylate (2-HEA), le 3-hydroxypropyl acrylate ; les (méth)acrylamides d'alkyle en C₁-C₄ hydroxylés, tels que le N-(2-hydroxyéthyl) méthacrylamide, le N-(2-hydroxyéthyl) acrylamide, le N-(3-hydroxypropyl) acrylamide, le N-(2,3-dihydroxypropyl) acrylamide ; et leurs mélanges. On peut encore citer l'alcool allylique, le glycérol monoallyl éther, le 3-méthyl-3-butèn-1-ol, les précurseurs de l'alcool vinylique et leurs équivalents, tel que l'acétate de vinyle.
Lorsqu'il(s) est (sont) présent(s), le ou les monomères vinyliques non ioniques hydroxylés représentent généralement jusqu'à 10% en poids du poids total du mélange de monomères. De préférence, le ou les monomères vinyliques non ioniques hydroxylés représentent de 0,01 à 10% en poids, mieux de 1 à 8%, et encore de 1 à 5% en poids par rapport au poids total du mélange de monomères.

Le ou les polymères cationiques utilisés (i) dans la composition selon l'invention sont préparés à partir d'un mélange de monomères qui peut comprendre un ou plusieurs monomères réticulants permettant d'introduire des ramifications et de contrôler la masse moléculaire.
Des agents réticulants poly-insaturés utilisables sont bien connus dans l'état de la technique. Des composés mono-insaturés présentant un groupe réactif capable de réticuler un copolymère formé avant, pendant ou après la polymérisation peuvent aussi être utilisés. D'autres monomères réticulant utilisables peuvent être des monomères polyfonctionnels comprenant des groupes réactifs multiples tels que des groupes époxydes, isocyanates et des groupes silanes hydrolysables. De nombreux composés poly-insaturés peuvent être utilisés pour générer un réseau tri-dimensionnel partiellement ou substantiellement réticulé.
Des exemples de monomères réticulants polyinsaturés utilisables sont par exemple les monomères aromatiques polyinsaturés, tel que le divinylbenzène, le divinyl naphtylène, et le trivinylbenzène ; les monomères alicycliques polyinsaturés, tel que le 1,2,4-trivinylcyclohexane ; les esters difonctionnels de l'acide phthalique, tel que le diallyl phthalate ; les monomères aliphatiques polyinsaturés, tels que les diènes, les triènes et les tétraènes, notamment l'isoprène, le butadiène, le 1,5-hexadiène, le 1,5,9-décatriène, le 1,9-décadiène, et le 1,5 heptadiène.
D'autres monomères réticulants polyinsaturés utilisables sont par exemple les éthers polyalcényles, tels que le triallyl pentaérythritol, le diallyl pentaérythritol, le diallyl saccharose, l'octaallyl saccharose et le triméthylolpropane diallyl éther ; les esters polyinsaturés de polyalcools ou de polyacides, tel que le 1,6-hexanediol di(méth)acrylate, le tétraméthylène tri(méth)acrylate, l'allyl acrylate, le diallyl itaconate, le diallyl fumarate, le diallyl maléate, le triméthylolpropane tri(méth)acrylate, le triméthylolpropane di(méth)acrylate et le polyéthylène glycol di(méth)acrylate, les alkylène bisacrylamides, tel que le méthylène bisacrylamide, le propylène bisacrylamide ; les dérivés hydroxylés et carboxylés du méthylène bisacrylamide, tel que le N,N'-bisméthylol méthylène bisacrylamide ; les polyéthylèneglycol di(méth)acrylates, tels que l'éthylèneglycol di(méth)acrylate, le diiéthylèneglycol di(méth)acrylate, le triéthylèneglycol di(méth)acrylate, les silanes polyinsaturés tels que le diméthyldivinylsilane, le méthyltrivinylsilane, l'allyldiméthylvinylsilane, le diallyldiméthylsilane et le tétravinylsilane, les stannanes polyinsaturés, tel que le tétraallyl étain et le diallyldiméthyl étain.
Des monomères réticulants monoinsaturés utilisables et portant un groupe réactif peuvent être les N-méthylolacrylamides ; les N-alkoxy(méth)acrylamides, où le groupe alkoxy est un groupe C₁-C₁₈ ; et les silanes hydrolysables insaturés tel que triéthoxyvinylsilane, le tris-isopropoxyvinylsilane, et le 3-triéthoxysilylpropyl méthacrylate.
Des monomères réticulants polyfonctionnels utilisables et comprenant plusieurs groupes réactifs peuvent être par exemple les silanes hydrolysables tel que l'éthyltriéthoxysilane et l'éthyltriméthoxysilane ; les silanes hydrolysables époxylés, tel que le 2-(3,4-époxycyclohexyl)éthyltriéthoxysilane et le 3-glycidoxypropyltriméthyoxysilane ; les polyisocyanates, tels que le 1,4-diisocyanatobutane, le 1,6-diisocyanatohexane, le 1,4-phénylènediisocyanate, et le 4,4'-oxybis(phénylisocyanate) ; les époxides insaturés, tel que le glycidyl méthacrylate et l'allylglycidyl éther ; les polyépoxides, tel que le diglycidyl éther, le 1,2,5,6-diépoxyhexane, et l'éthylèneglycoldiglycidyl éther.
Des monomères réticulants polyinsaturés particulièrement utilisables sont les polyols éthoxylés, tels les diols, les triols et les bis-phénols, éthoxylés avec 2 à 100 moles d'oxyde d'éthylène par mole de groupe fonctionnel hydroxyle et terminés par un groupe insaturé polymérisable tel que un vinyl éther, un allyl éther, un ester acrylate ou un ester méthacrylate. De tels monomères réticulants peuvent être par exemple le diméthacrylate éthoxylé de biphénol A, le diméthacrylate éthoxylé de biphénol F, et le triméthylol propane triméthacrylate éthoxylé.
D'autres monomères réticulants éthoxylés utilisables dans la présente invention sont par exemple les agents réticulants dérivés des polyols éthoxylés divulgués dans le brevet US 6 140 435.
Des exemples particulièrement préférés de monomères réticulants sont des esters acrylates et méthacrylates de polyols ayant au moins deux groupes ester acrylate ou méthacrylate, tel que le triméthylolpropane triacrylate (TMPTA), le triméthylolpropane diméthacrylate, le triéthylène glycol diméthacrylate (TEGDMA), et le diméthacrylate de bisphénol A éthoxylé (30) (EOBDMA).
Lorsqu'il(s) est (sont) présent(s), le ou les monomères réticulants représentent de préférence au plus 5% en poids par rapport au poids du mélange de monomères. Selon un mode de réalisation préféré, les monomères réticulants sont présents à une teneur variant de 0,001 à 5% en poids, de préférence de 0,05 à 2% en poids, mieux de 0,1 à 1% en poids par rapport au poids total du mélange de monomères.

Le mélange de monomères peut comprendre un ou plusieurs agents de transfert de chaîne. Les agents de transferts de chaîne sont des composés bien connus de l'état de la technique.
On peut citer en particulier les composés thiolés, les composés disulfures, tels que les C₁-C₁₈ mercaptans, les acides mercaptocarboxyliques, les esters d'acides mercaptocarboxyliques, les thioesters, les C₁-C₁₈ alkyl disulfures, les aryldisulfures, les thiols polyfonctionnels ; les phosphites et hypophosphites ; les composés haloalkyl, tel que le tétrachlorure de carbone, le bromotrichlorométhane ; et les agents de transfert de chaîne insaturés, tel que l'alphaméthylstyrène.
Les thiols polyfonctionnels sont par exemple les thiols trifonctionnels, tel que le triméthylolpropane-tris-(3-mercaptopropionate), les thiols tétrafonctionnels, tel que le pentaérythritol-tétra-(3-mercaptopropionate), le pentaérythritol-tétra-(thioglycolate) et le pentaérythritol-tétra(thiolactate) ; les thiols hexafonctionnels, tel que le pentaérytritol-hexa-(thioglyconate).
De façon alternative, le ou les agents de transfert de chaîne peuvent être des agents de transfert de chaîne catalytiques qui réduisent le poids moléculaire des polymères d'addition lors de la polymérisation par radicaux libres des monomères vinyliques. On peut citer par exemple les complexes de cobalt, notamment les chélates de cobalt (II). Les agents de transfert de chaîne catalytiques peuvent souvent être utilisés à des concentrations faibles par rapport aux agents de transfert de chaîne thiolés.
De façon particulièrement préférée, on peut citer à titre d'agent de transfert de chaîne l'octyl mercaptan, le n-dodécyle mercaptan, le t-dodécyle mercaptan, l'hexadécyle mercaptan, l'octadécyle mercaptan (ODM), l'isooctyl 3-mercaptopropionate (IMP), le butyl 3-mercaptopropionate, l'acide 3-mercaptopropionique, le butyl thioglycolate, l'isooctyl thioglycolate, le dodécyle thioglycolate.
Lorsqu'il(s) est (sont) présents, le ou les agents de transferts de chaîne sont ajoutés au mélange de monomères de préférence jusqu'à 10% en poids par rapport au poids total du mélange de monomère. De préférence, le ou les agents de transfert de chaîne représentent de 0,1% à 5 % en poids par rapport au poids total de monomères.

Le mélange de monomères permettant la préparation du polymère cationique (i) utilisé dans la composition selon l'invention peut comprendre un ou plusieurs stabilisateurs polymériques pour l'obtention de dispersions ou d'émulsions stables. De préférence, les polymères sont solubles dans l'eau. On peut citer par exemple les polymères synthétiques, tels que les alcools polyvinyliques, les acétates polyvinyliques partiellement hydrolysés, la polyvinylpyrrolidone, les polyacrylamides, les polyméthacrylamides, les polymères d'addition carboxylés, les polyalkyles vinyle éthers ; les polymères naturels hydrosolubles, tels que la gélatine, les peptines, les alginates, la caséine; les polymères naturels modifiés, tels que la méthylcellulose, l'hydroxypropylcellulose, la carboxyméthylcellulose, les hydroxyéthylcelluloses allyliques.
Les stabilisateurs polymériques sont utilisés en une quantité au plus égale à 2 % en poids par rapport au poids total de l'émulsion, de préférence en une quantité comprise 0,0001 et 1 % en poids, mieux entre 0,01 et 0,5 % en poids par rapport au poids du mélange de monomères.

Selon un mode de réalisation préféré, le mélange de monomères comprend, par rapport au poids total du mélange de monomères :
a) de 10 à 70 % en poids du ou des monomères vinyliques substitués par un ou plusieurs groupes amino,
b) de 20 à 80 % en poids du ou des monomères vinyliques non ioniques hydrophobes,
c) de 0,001 à 25 % en poids du ou des monomères vinyliques associatifs,
d) de 0 à 25 % en poids du ou des monomères tensioactifs vinyliques semi-hydrophobes,
e) de 0 à 10 % en poids d'un ou plusieurs monomères vinyliques non ioniques hydroxylés,
f) de 0 à 5% en poids d'un ou plusieurs monomères réticulants,
g) de 0 à 10 % en poids d'un ou plusieurs agents de transfert de chaîne, et
h) de 0 à 2 % en poids d'un ou plusieurs stabilisateurs polymériques.

De façon encore plus préférée, le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10 % en poids du ou des agents de transfert de chaîne,
h) de 0 à 2 % en poids du ou des stabilisateurs polymériques.

Selon un mode de réalisation particulièrement préféré, le mélange de monomères permettant la préparation du polymère cationique (i) utilisé dans la composition selon l'invention comprend, par rapport au poids total du mélange de monomères :
a) de 20 à 50 % en poids d'un ou plusieurs monomères vinyliques substitués par un ou plusieurs groupes amino choisis parmi :
   - le (méth)acrylate de 3-(N,N-diméthylamino)propyle,
   - le N'-(3-N,N-diméthylamino)propyl(méth)acrylamide,
   - le (méth)acrylate de 2-(N,N-diméthylamino)éthyle,
   - le (méth)acrylate de 2-(N,N-diéthylamino)éthyle,
   - le (méth)acrylate de 2-(tert-butylamino)éthyle,
   - le 2-(N,N-diméthylamino)propyl (méth)acrylamide, et
   - l'acrylate de 2-(N,N-diméthylamino)néopentyle,
b) de 50 à 65 % en poids d'un ou plusieurs monomères vinyliques non ioniques hydrophobes choisis parmi les esters d'acide acrylique et d'alkyle en C₁-C₃₀, les esters de l'acide méthacrylique et d'alkyle en C₁-C₃₀, et leurs mélanges,
c) de 0,1 à 10 % en poids d'un ou plusieurs monomères vinyliques associatifs choisis parmi les méthacrylates polyéthoxylés de cétyle, les méthacrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6 (1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges,
d) de 0,1 à 10 % en poids d'un ou plusieurs monomères tensioactifs vinyliques semi-hydrophobes ayant l'une des formules suivantes :

   CH₂=CH-O(CH₂)ₐO(C₃H₆O)_{b}(C₂H₄O)_{c}H ou

   CH₂=CHCH₂O(C₃H₆O)_{d}(C₂H₄O)ₑH;

   où
   a est égal à 2, 3, ou 4 ;
   b est un entier variant de 1 à 10 ;
   c est un entier variant de 5 à 50 ;
   d est un entier variant de 1 à 10 ; et
   e est un entier variant de 5 à 50.
e) jusqu'à 10 % en poids d'un ou plusieurs monomères vinyliques non ioniques hydroxylés,
f) jusqu'à 5% en poids d'un ou plusieurs monomères réticulants,
g) jusqu'à 10 % en poids d'un ou plusieurs agents de transfert de chaîne, et
h) jusqu'à 2 % en poids d'un ou plusieurs stabilisateurs polymériques.

Les polymères cationiques (i) préférés selon l'invention sont des polymères issus de la polymérisation du mélange de monomères suivants :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

Parmi les polymères cationiques (i) utilisés dans la composition selon l'invention, on peut notamment citer le composé commercialisé par la société NOVEON sous la dénomination CARBOPOL AQUA CC POLYMER et qui correspond à la dénomination INCI POLYACRYLATE-1 CROSSPOLYMER.
Le POLYACRYLATE-1 CROSSPOLYMER est le produit de la polymérisation d'un mélange de monomères comprenant (ou constitué de):
- un méthacrylate de di(alkyl en C₁-C₄) amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé (20-25 moles de motif oxyde d'éthylène),
- un allyl éther de polyéthylèneglycol/polypropylèneglycol 30/5,
- un méthacrylate d'hydroxy(alkyle en C₂-C₆), et
- un diméthacrylate d'éthylèneglycol.

Le ou les polymères cationiques (i) utilisés dans les compositions selon l'invention représentent généralement de 0,01 à 10% en poids, de préférence de 0,05 à 5% en poids, et mieux de 0,1 à 1% en poids par rapport au poids total de la composition.

Le ou les polymères cationiques (i) utilisés dans la composition selon l'invention peuvent être préparés par des techniques de polymérisation conventionnelles, telles que la polymérisation en émulsion, comme cela est bien connu dans le domaine des polymères. La polymérisation peut être effectuée par simple procédé discontinu, par procédé par addition contrôlée, ou bien la réaction peut être initiée dans un petit réacteur puis alors la masse des monomères peut être ajoutée de façon contrôlée dans le réacteur (procédé par ensemencement). Généralement, la polymérisation est menée à une température de réaction comprise entre 20 et 80°C, même si des températures supérieures ou inférieures peuvent être utilisées. Pour faciliter l'émulsification du mélange de monomères, la polymérisation par émulsion est effectuée en présence d'un tensioactif présent en une quantité variant de 1 à 10 % en poids, de préférence de 3 à 8% en poids, mieux de 5 à 7 % en poids, par rapport au poids total de l'émulsion. Le milieu de réaction de polymérisation par émulsion comprend également un ou plusieurs initiateurs radicalaires, de préférence en une quantité variante de 0,01 à 3 % en poids par rapport au poids total du mélange de monomères. La polymérisation peut être réalisée dans un milieu aqueux ou bien hydroalcoolique à un pH neutre ou faiblement alcalin.
Dans une polymérisation typique, le mélange de monomères est ajouté sous agitation à une solution de tensioactifs émulsifiants, tel qu'un tensioactif non ionique, de préférence un éthoxylate d'alcool linéaire ou ramifié, ou un mélange de tensioactifs non ioniques et anioniques, tels que des sulfates d'alcool gras ou des alkyles sulfonates d'alcool gras, dans une quantité adaptée d'eau, dans un réacteur adapté, pour préparer l'émulsion de monomères. L'émulsion est désoxygénée au moyen de toute méthode connue, puis la réaction de polymérisation est initiée en ajoutant un catalyseur de polymérisation (initiateur) tel que le persulfate de sodium, ou tout autre catalyseur de polymérisation par addition adaptée, comme cela est bien connu dans le domaine des polymères. La réaction est agitée jusqu'à ce que la polymérisation soit complète, généralement pendant une durée variant de 4 heures à 16 heures. L'émulsion de monomères peut être chauffée à une température comprise entre 20 et 80°C avant l'addition de l'initiateur, si cela est souhaité. La quantité de monomères n'ayant pas réagi peut être éliminée par addition d'une quantité supplémentaire de catalyseur. L'émulsion de polymère obtenue peut être retirée du réacteur et emballée pour être stockée ou utilisée. De façon optionnelle, le pH ou d'autres caractéristiques physiques ou chimiques de l'émulsion peuvent être ajustés avant de retirer l'émulsion du réacteur. Généralement, l'émulsion produite a une teneur totale en solides qui varie entre 10 et 40 % en poids. Généralement, la quantité totale de polymères dans l'émulsion obtenue varie entre 15 et 35% en poids, en général au plus 25 % en poids.
Des tensioactifs adaptés pour faciliter la polymérisation par émulsion peuvent être des tensioactifs non ioniques, anioniques, amphotères ou cationiques, ou leurs mélanges. Le plus souvent, des tensioactifs non ioniques
ou anioniques, ou leurs mélanges sont utilisés.

Tous types de tensioactifs non ioniques, anioniques, amphotères ou cationiques classiquement utilisés dans les polymérisations en l'émulsion peuvent être utilisés.
La polymérisation peut être effectuée en présence d'un ou plusieurs initiateurs de radicaux libres. Ceux-ci peuvent être choisis parmi les composés persulfates inorganiques insolubles, tels que le persulfate d'ammonium, le persulfate de potassium, le persulfate de sodium ; les peroxydes tels que le peroxyde d'hydrogène, le peroxyde de benzoyle, le peroxyde d'acétyle, et le peroxyde de lauryle ; les hydroperoxydes organiques, tels que l'hydroperoxyde de cumen et l'hydroperoxyde de t-butyle ; les peracides organiques, tel que l'acide peracétique ; et les agents producteurs de radicaux libres solubles dans l'huile, tels que 2,2'-azobisisobutyronitrile, et leurs mélanges. Les peroxydes et les peracides peuvent être éventuellement activés avec des agents réducteurs, tel que le bisulfite de sodium ou l'acide ascorbique, les métaux de transition, l'hydrazine. Des initiateurs de radicaux libres particulièrement adaptés sont les initiateurs de polymérisation azoïque solubles dans l'eau, tels que les composés 2,2'-azobis(tert-alkyl) ayant un substituant hydrosolubilisant sur le groupe alkyle. Des catalyseurs de polymérisation azoïque préférés sont les initiateurs de radicaux libres VAZO^{®}, commercialisés par la société DuPont, tel que le VAZO^{®}44 (2,2'-azobis(2-4,5-dihydroimidazolyl)propane), le VAZO^{®}56 (2,2'-azobis(2-méthylpropio-namidine)dihydrochlorure), et le VAZO^{®}68 (4,4'-azobis(acide 4-cyanovalérique)).
Les silicones utilisables conformément à l'invention sont en particulier insolubles dans la composition et peuvent se présenter sous forme d'huiles, de cires, de résines ou de gommes.

Au sens de l'invention, on entend désigner par silicone, en conformité avec l'acceptation générale, tous polymères ou oligomères organosiliciés à structure linéaire ou cyclique, ramifiée ou réticulée, de poids moléculaire variable, obtenus par polymérisation et/ou par polycondensation de silanes convenablement fonctionnalisés, et constitués pour l'essentiel par une répétition de motifs principaux dans lesquels les atomes de silicium sont reliés entre eux par des atomes d'oxygène (liaison siloxane -Si-O-Si-), des radicaux hydrocarbonés éventuellement substitués, étant directement liés par l'intermédiaire d'un atome de carbone sur lesdits atomes de silicium. Les radicaux hydrocarbonés les plus courants sont les radicaux alkyles, notamment en C₁-C₁₀, et en particulier méthyle, les radicaux fluoroalkyles dont la partie alkyle est en C₁-C₁₀, les radicaux aryles et en particulier phényle.

Par silicone aminé, on entend au sens de la présente invention, toute silicone comportant une ou plusieurs fonctions amine primaire, secondaire, tertiaire ou un ou plusieurs groupements ammonium quaternaire.

Les silicones aminées utilisées dans la composition cosmétique selon la présente invention sont de préférence choisies parmi :
(a) les composés répondant à la formule (VI) suivante :

   (R¹)ₐ(T)₃₋ₐ-Si[OSi(T)₂]ₙ-[OSi(T)_{b}(R¹)_{2-b}]ₘ-OSi(T)₃₋ₐ-(R¹)ₐ (VI)

   dans laquelle,
   T est un atome d'hydrogène, ou un radical phényle, hydroxyle (-OH), ou alkyle en C₁-C₈, et de préférence méthyle ou alcoxy en C₁-C₈, de préférence méthoxy,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R₁ est un radical monovalent de formule -C_{q}H_{2q}L dans laquelle q est un nombre de 2 à 8 et L est un groupement aminé éventuellement quaternisé choisi parmi les groupements :
   -N(R²)-CH₂-CH₂-N(R²)₂ ;
   -N(R²)₂ ; -N⁺(R²)₃ Q⁻ ;
   -N⁺(R²) (H)₂ Q- ;
   -N⁺(R²)₂HQ- ;
   -N(R²)-CH₂-CH₂-N⁺(R²)(H)₂ Q-,
   dans lesquels R² peut désigner un atome d'hydrogène, un phényle, un benzyle, ou un radical hydrocarboné saturé monovalent, par exemple un radical alkyle en C₁-C₂₀, et Q- représente un ion halogénure tel que par exemple fluorure, chlorure, bromure ou iodure.
   En particulier, les silicones aminées correspondant à la définition de la formule (VI) sont choisies parmi les composés correspondant à la formule suivante : 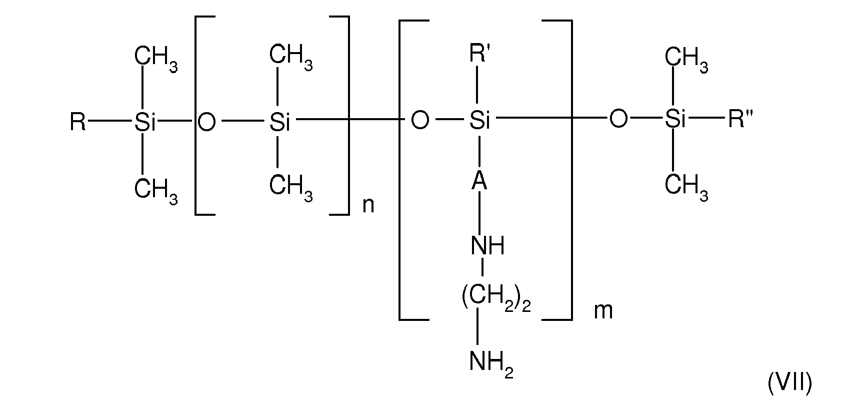 dans laquelle R, R', R", identiques ou différents, désignent un radical alkyle en C₁-C₄, de préférence CH₃ ; un radical alcoxy en C₁-C₄, de préférence méthoxy ; ou OH ; A représente un radical alkylène, linéaire ou ramifié, en C₃-C₈, de préférence en C₃-C₆; m et n sont des nombres entiers dépendant du poids moléculaire et dont la somme est comprise entre 1 et 2000.
   Selon une première possibilité, R, R', R", identiques ou différents, représentent un radical alkyle en C₁-C₄ ou hydroxyle, A représente un radical alkylène en C₃ et m et n sont tels que la masse moléculaire moyenne en poids du composé est comprise entre 5 000 et 500 000 environ. Les composés de ce type sont dénommés dans le dictionnaire CTFA, "amodiméthicone".
   Selon une deuxième possibilité, R, R', R", identiques ou différents, représentent un radical alcoxy en C₁-C₄ ou hydroxyle, l'un au moins des radicaux R ou R" est un radical alcoxy et A représente un radical alkylène en C₃. Le rapport molaire hydroxy / alcoxy est de préférence compris entre 0,2/1 et 0,4/1 et avantageusement égal à 0,3/1. Par ailleurs, m et n sont tels que la masse moléculaire moyenne en poids du composé est comprise entre 2000 et 10⁶. Plus particulièrement, n est compris entre 0 et 999 et m est compris entre 1 et 1000, la somme de n et m étant comprise entre 1 et 1000.
   Dans cette catégorie de composés, on peut citer entre autres, le produit Belsil®ADM 652, commercialisée par Wacker.
   Selon une troisième possibilité, R, R", différents, représentent un radical alcoxy en C₁-C₄ ou hydroxyle, l'un au moins des radicaux R, R" est un radical alcoxy, R' représente un radical méthyle et A représente un radical alkylène en C₃. Le rapport molaire hydroxy / alcoxy est de préférence compris entre 1/0,8 et 1/1,1, et avantageusement est égal à 1/0,95. Par ailleurs, m et n sont tels que la masse moléculaire moyenne en poids du composé est comprise entre 2000 et 200000. Plus particulièrement, n est compris entre 0 et 999 et m est compris entre 1 et 1000, la somme de n et m étant comprise entre 1 et 1000.
   Plus particulièrement, on peut citer le produit FluidWR® 1300, commercialisé par Wacker.
   Selon une quatrième possibilité, R, R" représentent un radical hydroxyle, R' représente un radical méthyle et A est un radical alkylène, en C₄-C₈, de préférence en C₄. Par ailleurs, m et n sont tels que la masse moléculaire moyenne en poids du composé est comprise entre 2000 et 10⁶. Plus particulièrement, n est compris entre 0 et 1999 et m est compris entre 1 et 2000, la somme de n et m étant comprise entre 1 et 2000.
   Un produit de ce type est notamment commercialisé sous la dénomination DC28299 par Dow Corning.
   Notons que la masse moléculaire de ces silicones est déterminée par chromatographie par perméation de gel (température ambiante, étalon polystyrène ; colonnes µ styragem ; éluant THF ; débit de 1 mm/m ; on injecte 200 µl d'une solution à 0,5 % en poids de silicone dans le THF et l'on effectue la détection par réfractométrie et UV-métrie).
   Un produit correspondant à la définition de la formule (VI) est en particulier le polymère dénommé dans le dictionnaire CTFA "triméthylsilylamodiméthicone", répondant à la formule (VIII) suivante : 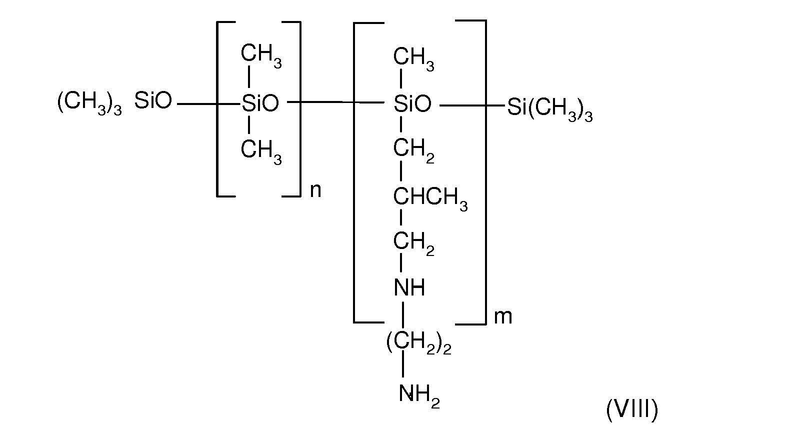 dans laquelle n et m ont les significations données ci-dessus conformément à la formule (VI).
   De tels composés sont décrits par exemple dans EP 95238; un composé de formule (VIII) est par exemple vendu sous la dénomination Q2-8220 par la société OSI.
(b) les composés répondant à la formule (IX) suivante : 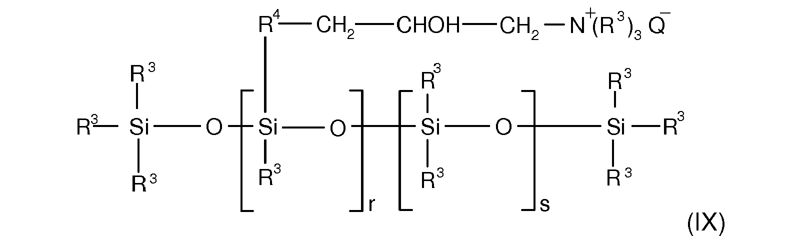 dans laquelle,
   R³ représente un radical hydrocarboné monovalent en C₁-C₁₈, et en particulier un radical alkyle en C₁-C₁₈, ou alcényle en C₂-C₁₈, par exemple méthyle ;
   R⁴ représente un radical hydrocarboné divalent, notamment un radical alkylène en C₁-C₁₈ ou un radical alkylèneoxy divalent en C₁-C₁₈, par exemple en C₁-C₈;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.
   De tels composés sont décrits plus particulièrement dans le brevet US 4185087.
   Un composé entrant dans cette classe est celui vendu par la Société Union Carbide sous la dénomination "Ucar Silicone ALE 56".
(c) les silicones ammonium quaternaire notamment de formule (X) : 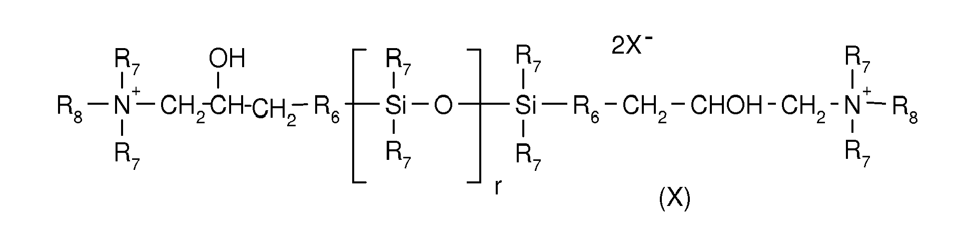 dans laquelle :
   R₇, identiques ou différents, représentent un radical hydrocarboné monovalent ayant de 1 à 18 atomes de carbone, et en particulier un radical alkyle en C₁-C₁₈, un radical alcényle en C₂-C₁₈ ou un cycle comprenant 5 ou 6 atomes de carbone, par exemple méthyle ;
   R₆ représente un radical hydrocarboné divalent, notamment un radical alkylène en C₁-C₁₈ ou un radical alkylèneoxy divalent en C₁-C₁₈, par exemple en C₁-C₈ relié au Si par une liaison SiC;
   R₈, identiques ou différents, représentent un atome d'hydrogène, un radical hydrocarboné monovalent ayant de 1 à 18 atomes de carbone, et en particulier un radical alkyle en C₁-C₁₈, un radical alcényle en C₂-C₁₈, un radical -R₆-NHCOR₇ ;
   X- est un anion tel qu'un ion halogénure, notamment chlorure ou un sel d'acide organique (acétate ...);
   r représente une valeur statistique moyenne de 2 à 200 et en particulier de 5 à 100 ;
      Ces silicones sont par exemple décrites dans la demande EP-A-0530974.
(d) les silicones aminées de formule (XI) : 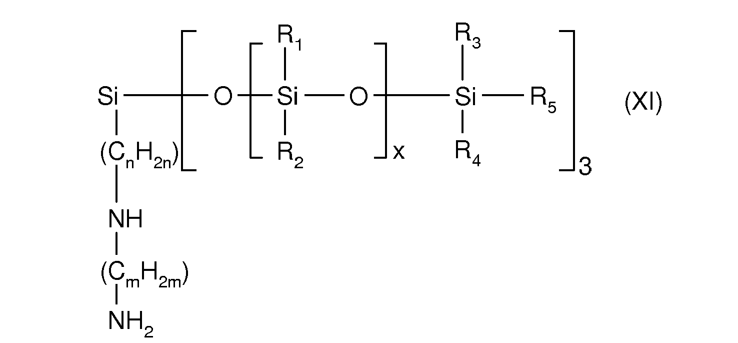 dans laquelle :
   - R₁, R₂, R₃ et R₄, identiques ou différents, désignent un radical alkyle en C₁-C₄ ou un groupement phényle,
   - R₅ désigne un radical alkyle en C₁-C₄ ou un groupement hydroxyle,
   - n est un entier variant de 1 à 5,
   - m est un entier variant de 1 à 5,
   et dans laquelle x varie de 2 à 500, de préférence de 5 à 100.

Les silicones particulièrement préférées conformément à l'invention sont les polysiloxanes à groupements aminés tels que les amodiméthicones ou les triméthylsilylamodiméthicones (CTFA 4ème édition 1997), et encore plus particulièrement les silicones à groupements ammonium quaternaire.
Lorsque ces composés sont mis en oeuvre, une forme de réalisation particulièrement intéressante est leur utilisation conjointe avec des agents de surface cationiques et/ou non ioniques.

A titre exemple, on peut utiliser le produit vendu sous la dénomination "Emulsion Cationique DC 929" par la Société Dow Corning, qui comprend, outre l'amodiméthicone, un agent de surface cationique comprenant un mélange de produits répondant à la formule : 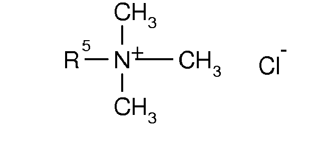 dans laquelle, R⁵ désigne des radicaux alcényle et/ou alcoyle en C₁₄-C₂₂ dérivés des acides gras du suif, et connu sous la dénomination CTFA "tallowtrimonium chloride",en association avec un agent de surface non ionique de formule :

C₉H₁₉-C₆H₄-(OC₂H₄)₁₀-OH,

connu sous la dénomination CTFA "Nonoxynol 10".

On peut également utiliser par exemple le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".
Un autre produit commercial utilisable selon l'invention est le produit vendu sous la dénomination "Dow Corning Q2 7224" par la Société Dow Corning, comportant en association le triméthylsilylamodiméthicone de formule (C) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

Selon l'invention, toutes les silicones peuvent également être utilisées sous forme d'émulsions ou de microémulsions.

Selon l'invention, la ou les silicones aminées peuvent représenter de 0,001 % à 20 % en poids, de préférence de 0,01 % à 10% en poids et plus particulièrement de 0,1 à 3% en poids par rapport au poids total de la composition finale.

La composition cosmétique selon l'invention comprend un ou plusieurs polymères cationiques (iii) dont la densité de charge cationique est supérieure à 4 milliéquivalents par gramme (meq/g), de préférence supérieure ou égale à 5 milliéquivalents par gramme (meq/g), de préférence allant de 5 à 20 méq/g et plus particulièrement de 5,5 à 10 meq/g.

La densité de charge cationique d'un polymère correspond au nombre de moles de charges cationiques par unité de masse de polymère dans les conditions où celui-ci est totalement ionisé. Elle peut être déterminée par calcul si on connaît la structure du polymère, c'est-à-dire la structure des monomères constituant le polymère et leur proportion molaire ou pondérale. Elle peut être aussi déterminée expérimentalement par la méthode Kjeldahl, généralement à un pH d'environ 7 à température ambiante.

Les polymères cationiques ayant une densité de charge cationique supérieure à 4 méq/g, utilisables conformément à la présente invention, peuvent être choisis parmi tous ceux déjà connus en soi comme améliorant les propriétés cosmétiques des cheveux traités par des compositions, à savoir notamment ceux décrits dans la demande de brevet EP-A-0 337 354 et dans les demandes de brevets français FR-A-2 270 846, 2 383 660, 2 598 611,2 470 596 et 2 519 863.
De manière générale, au sens de la présente invention, l'expression "polymère cationique" désigne tout polymère comprenant des groupes cationiques et/ou des groupes ionisables en groupes cationiques.

Les polymères cationiques sont choisis parmi ceux qui comprennent des motifs comportant des groupements amine primaires, secondaires, tertiaires et/ou quaternaires pouvant soit faire partie de la chaîne principale polymère, soit être portés par un substituant latéral directement relié à celle-ci.

Les polymères cationiques utilisés ont généralement une masse molaire moyenne en nombre comprise entre 500 et 5.10⁶ environ, et de préférence comprise entre 10³ et 3.10⁶ environ.

Parmi les polymères cationiques, on peut citer plus particulièrement les polymères du type polyamine, polyaminoamide et polyammonium quaternaire. Ce sont des produits connus.

Parmi ces polymères, on peut citer :
(1) les homopolymères ou copolymères dérivés d'esters ou d'amides acryliques ou méthacryliques et comportant au moins un des motifs de formules suivantes: 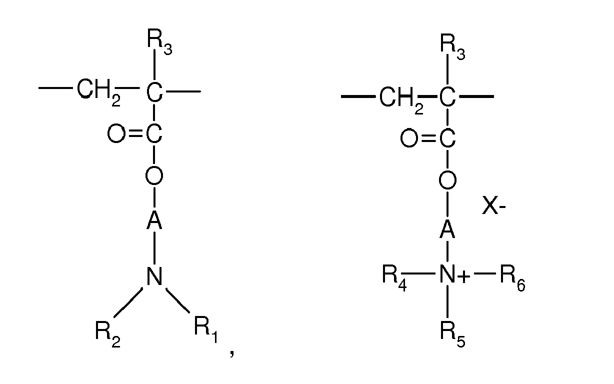 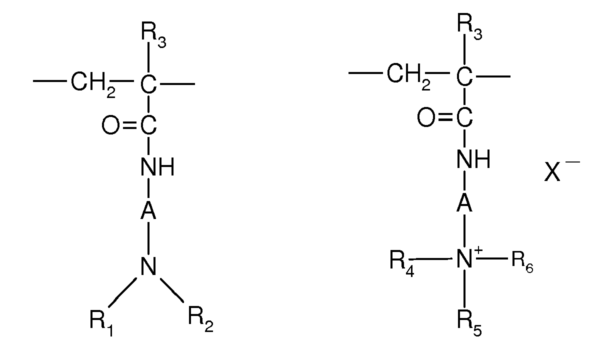 dans lesquelles:
   R₃ , identiques ou différents, désignent un atome d'hydrogène ou un radical CH₃;
   A, identiques ou différents, représentent un groupe alkyle, linéaire ou ramifié, de 1 à 6 atomes de carbone, de préférence 2 ou 3 atomes de carbone ou un groupe hydroxyalkyle de 1 à 4 atomes de carbone ;
   R₄, R₅, R₆, identiques ou différents, représentent un groupe alkyle ayant de 1 à 18 atomes de carbone ou un radical benzyle et de préférence un groupe alkyle ayant de 1 à 6 atomes de carbone;
   R₁ et R₂ , identiques ou différents, représentent hydrogène ou un groupe alkyle ayant de 1 à 6 atomes de carbone et de préférence méthyle ou éthyle;
   X désigne un anion dérivé d'un acide minéral ou organique en particulier un anion méthosulfate ou un halogénure, notamment chlorure ou bromure.
   Les copolymères de la famille (1) peuvent comprendre en outre un ou plusieurs motifs dérivant de comonomères pouvant être choisis dans la famille des acrylamides, méthacrylamides, diacétones acrylamides, acrylamides et méthacrylamides substitués sur l'azote par des alkyles inférieurs (C1-C4), des acides acryliques ou méthacryliques ou leurs esters, des vinyllactames tels que la vinylpyrrolidone ou le vinylcaprolactame, des esters vinyliques.
   Ainsi, parmi ces copolymères de la famille (1), on peut citer :
   - les copolymères d'acrylamide et de diméthylaminoéthyl méthacrylate quaternisé au sulfate de diméthyle ou avec un halogénure de diméthyle
   - les copolymères d'acrylamide et de chlorure de méthacryloyloxyéthyl-triméthylammonium décrit par exemple dans la demande de brevet EP-A-080976
   - le copolymères d'acrylamide et de méthosulfate de méthacryloyloxy-éthyltriméthylammonium,
   - les copolymères vinylpyrrolidone / acrylate ou méthacrylate de dialkylaminoalkyle quaternisés ou non. Ces polymères sont décrits en détail dans les brevets français 2.077.143 et 2.393.573,
   - les terpolymères méthacrylate de diméthyl amino éthyle vinylcaprolactame/ vinylpyrrolidone,
   - les copolymères vinylpyrrolidone / méthacrylamidopropyl dimethylamine.
   - les copolymères vinylpyrrolidone / méthacrylamide de diméthylaminopropyle quaternisé,
   - et les polymères réticulés de sels de méthacryloyloxyalkyl(C₁-C₄) trialkyl(C₁-C₄)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion comprenant 50 % en poids dudit copolymère dans de l'huile minérale. Cette dispersion est commercialisée sous le nom de « SALCARE^{®} SC 92 » par la Société CIBA. On peut également utiliser un homopolymère réticulé du chlorure de méthacryloyloxyéthyl triméthylammonium comprenant environ 50 % en poids de l'homopolymère dans de l'huile minérale ou dans un ester liquide. Ces dispersions sont commercialisées sous les noms de « SALCARE^{®} SC 95 » et « SALCARE^{®} SC 96 » par la Société CIBA.
(2) les cyclopolymères d'alkyl diallyl amine ou de dialkyl diallyl ammonium tels que les homopolymères ou copolymères comportant comme constituant principal de la chaîne des motifs répondant aux formules (XII) ou (XIII) : 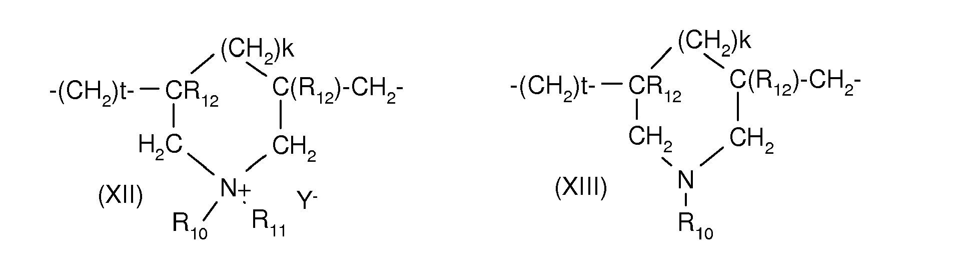 formules dans lesquelles k et t sont égaux à 0 ou 1, la somme k + t étant égale à 1 ; R₁₂ désigne un atome d'hydrogène ou un radical méthyle ; R₁₀ et R₁₁, indépendamment l'un de l'autre, désignent un groupement alkyle ayant de 1 à 6 atomes de carbone, un groupement hydroxyalkyle dans lequel le groupement alkyle a de préférence 1 à 5 atomes de carbone, un groupement amidoalkyle inférieur (C1-C4) ou R₁₀ et R₁₁ peuvent désigner conjointement avec l'atome d'azote auquel ils sont rattachés, des groupement hétérocycliques, tels que pipéridinyle ou morpholinyle ; Y⁻ est un anion tel que bromure, chlorure, acétate, borate, citrate, tartrate, bisulfate, bisulfite, sulfate, phosphate. Ces polymères sont notamment décrits dans le brevet français 2.080.759 et dans son certificat d'addition 2.190.406.
   R₁₀ et R₁₁, indépendamment l'un de l'autre, désignent de préférence un groupement alkyle ayant de 1 à 4 atomes de carbone.
   Parmi les polymères définis ci-dessus, on peut citer plus particulièrement les homopolymères de sels (par exemple chlorure) de diméthyldiallylammonium vendus notamment sous la dénomination "MERQUAT 100" par la société NALCO (et ses homologues de faibles masses molaires moyenne en poids) et les copolymères de chlorure de diallyldiméthylammonium et d'acrylamide.
(3) Les copolymères quaternaires de vinyllactame (vinylpyrrolidone et/ou vinylcaprolactame) et de vinylimidazole
(4) le polymère de diammonium quaternaire contenant des motifs récurrents répondant à la formule : 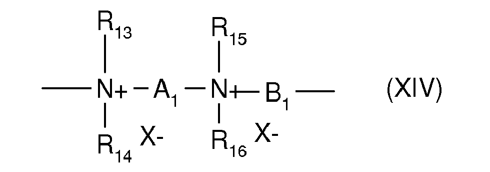 formule (XIV) dans laquelle :
   R₁₃, R₁₄, R₁₅ et R₁₆, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₁₃, R₁₄, R₁₅ et R₁₆, ensemble ou séparément, constituent avec les atomes d'azote auxquels ils sont rattachés des hétérocycles comprenant éventuellement un second hétéroatome autre que l'azote ou bien R_{13,} R₁₄, R₁₅ et R₁₆ représentent un radical alkyle en C₁-C₆ linéaire ou ramifié substitué par un groupement nitrile, ester, acyle, amide ou -CO-O-R₁₇-D ou -CO-NH-R₁₇-D où R₁₇ est un alkylène et D un groupement ammonium quaternaire ;
   A₁ et B₁ représentent des groupements polyméthyléniques comprenant de 2 à 20 atomes de carbone pouvant être linéaires ou ramifiés, saturés ou insaturés, et pouvant comprendre, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ désigne un anion dérivé d'un acide minéral ou organique;
   A₁, R₁₃ et R₁₅ peuvent former avec les deux atomes d'azote auxquels ils sont rattachés un cycle pipérazinique ; en outre si A₁ désigne un radical alkylène ou hydroxyalkylène linéaire ou ramifié, saturé ou insaturé, B₁ peut également désigner un groupement (CH₂)ₙ-CO-D-OC-(CH₂)p-
   dans lequel
   n et p, identiques ou différents, sont des nombres entiers variant de 2 à 20 environ
   D désigne :
   a) un reste de glycol de formule : -O-Z-O-, où Z désigne un radical hydrocarboné linéaire ou ramifié ou un groupement répondant à l'une des formules suivantes :

      -(CH₂-CH₂-O)ₓ -CH₂-CH₂-

      -[CH₂-CH(CH₃)-O]_{y}-CH₂-CH(CH₃)-

      où x et y désignent un nombre entier de 1 à 4, représentant un degré de polymérisation défini et unique ou un nombre quelconque de 1 à 4 représentant un degré de polymérisation moyen ;
   b) un reste de diamine bis-secondaire tel qu'un dérivé de pipérazine ;
   c) un reste de diamine bis-primaire de formule : -NH-Y-NH-, où Y désigne un radical hydrocarboné linéaire ou ramifié, ou bien le radical bivalent

      -CH₂-CH₂-S-S-CH₂-CH₂- ;
   d) un groupement uréylène de formule : -NH-CO-NH- ;
   De préférence, X⁻ est un anion tel que le chlorure ou le bromure.
   Ces polymères ont une masse moléculaire moyenne en nombre généralement comprise entre 1000 et 100000.
   Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.
   On peut utiliser plus particulièrement les polymères qui sont essentiellement constitués de motifs récurrents répondant à la formule : 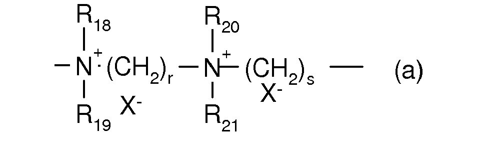 dans laquelle R₁₈, R₁₉, R₂₀ et R₂₁, identiques ou différents, désignent un radical alkyle ou hydroxyalkyle ayant de 1 à 4 atomes de carbone environ, r et s sont des nombres entiers variant de 2 à 20 environ et, X- est un anion dérivé d'un acide minéral ou organique.
   Un composé de formule (a) particulièrement préféré est celui pour lequel R₁₈, R₁₉, R₂₀ et R21, représentent un radical méthyle et r = 3, s = 6 et X = Cl, dénommé Hexadimethrine chloride selon la nomenclature INCI (CTFA).
(5) les polymères de polyammonium quaternaires constitués de motifs de formule (XV): 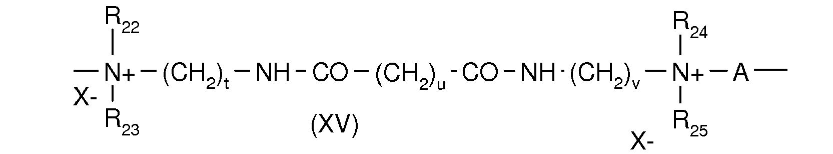 formule dans laquelle :
   R₂₂, R₂₃, R₂₄ et R₂₅, identiques ou différents, représentent un atome d'hydrogène ou un radical méthyle, éthyle, propyle, β-hydroxyéthyle, β-hydroxypropyle ou -CH₂CH₂(OCH₂CH₂)ₚOH,
   où p est égal à 0 ou à un nombre entier compris entre 1 et 6, sous réserve que R₂₂, R₂₃, R₂₄ et R₂₅ ne représentent pas simultanément un atome d'hydrogène,
   t et u, identiques ou différents, sont des nombres entiers compris entre 1 et 6,
   v est égal à 0 ou à un nombre entier compris entre 1 et 34,
   X⁻ désigne un anion tel qu'un halogènure,
   A désigne un radical d'un dihalogénure ou représente de préférence -CH₂-CH₂-O-CH₂-CH₂-.
   De tels composés sont notamment décrits dans la demande de brevet EP-A-122 324.
   On peut par exemple citer parmi ceux-ci, les produits "Mirapol® A 15", "Mirapol® AD1", "Mirapol® AZ1" et "Mirapol® 175" vendus par la société Miranol.

D'autres polymères cationiques utilisables dans le cadre de l'invention sont des polyalkylèneimines, en particulier des polyéthylèneimines, des polymères contenant des motifs vinylpyridine ou vinylpyridinium, des condensats de polyamines et d'épichlorhydrine, des polyuréylènes quaternaires et les dérivés de la chitine.

Parmi tous les polymères cationiques susceptibles d'être utilisés dans le cadre de la présente invention, on préfère mettre en oeuvre les cyclopolymères cationiques, en particulier les homopolymères de chlorure de diméthyldiallylammonium, vendus sous les dénominations « MERQUAT 100 » par la société NALCO (et ses homologues de faibles masses molaires moyenne en poids), les polyéthylèneimines et leurs mélanges.

Selon l'invention, le ou les polymères cationiques (iii) ayant une densité cationique supérieure à 4meq/g peuvent représenter de 0,001 % à 10 % en poids, de préférence de 0,005 % à 5 % en poids, et encore plus préférentiellement de 0,01 % à 3 % en poids, du poids total de la composition finale.

Selon un mode de réalisation particulier, les compositions selon l'invention comprennent en outre une ou plusieurs silicones non aminées et/ou un ou plusieurs autres agents bénéfiques pour les matières kératiniques en particulier les cheveux tels que notamment les esters d'acides carboxyliques en C1-C30 et d'alcools mono ou polyhydroxylés en C1-C30, les huiles végétales, animales, minérales ou de synthèse, les cires, les céramides, les pseudocéramides.

Les silicones additionnelles non aminées utilisables conformément à l'invention sont en particulier des polyorganosiloxanes insolubles dans la composition et peuvent se présenter sous forme d'huiles, de cires, de résines ou de gommes.

Ces organopolysiloxanes additionnels sont définis plus en détail dans l'ouvrage de Walter NOLL "Chemistry and Technology of Silicones" (1968) Academie Press. Elles peuvent être volatiles ou non volatiles.

Lorsqu'elles sont volatiles, les silicones additionnelles sont plus particulièrement choisies parmi celles possédant un point d'ébullition compris entre 60° C et 260° C, et plus particulièrement encore parmi :
(i) les silicones cycliques comportant de 3 à 7 atomes de silicium et de préférence 4 à 5. Il s'agit, par exemple, de l'octaméthylcyclotétrasiloxane commercialisé notamment sous le nom de "VOLATILE SILICONE 7207" par UNION CARBIDE ou "SILBIONE 70045 V 2" par RHODIA CHIMIE, le décaméthylcyclopentasiloxane commercialisé sous le nom de "VOLATILE SILICONE 7158" par UNION CARBIDE, "SILBIONE 70045 V 5" par RHODIA CHIMIE, ainsi que leurs mélanges.
   On peut également citer les cyclocopolymères du type diméthylsiloxanes méthylakylsiloxane, tel que la "SILICONE VOLATILE FZ 3109" commercialisée par la société UNION CARBIDE, de structure chimique : avec 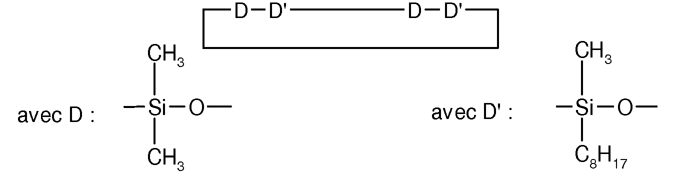 On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25° C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, P. 27-32 - TODD & BYERS "Volatile Silicone fluids for cosmetics".

Ces silicones additionnelles sont plus particulièrement choisies parmi les polyalkylsiloxanes parmi lesquels on peut citer principalement les polydiméthylsiloxanes à groupements terminaux triméthylsilyle ayant une viscosité de 5.10⁻⁶ à 2,5 m²/s à 25 °C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHODIA CHIMIE telles que par exemple l'huile 70 047 V 500 000 ;
- les huiles de la série MIRASIL commercialisées par la société RHODIA CHIMIE ;
- les huiles de la série 200 de la société DOW CORNING telles que plus particulièrement la DC200 de viscosité 60 000 Cst ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHODIA CHIMIE.

Dans cette classe de polyalkylsiloxanes, on peut également citer les produits commercialisés sous les dénominations "ABIL WAX 9800 et 9801" par la société GOLDSCHMIDT qui sont des polyalkyl (C₁-C₂₀) siloxanes.

Les gommes de silicone utilisables en tant qu'additif sont notamment des polydiorganosiloxanes ayant des masses molaires moyennes en nombre élevées comprises entre 200 000 et 1 000 000 utilisés seuls ou en mélange dans un solvant. Ce solvant peut être choisi parmi les silicones volatiles, les huiles polydiméthylsiloxanes (PDMS), les huiles poly-phénylméthylsiloxanes (PPMS), les isoparaffines, les polyisobutylènes, le chlorure de méthylène, le pentane, le dodécane, le tridécanes ou leurs mélanges.

On peut plus particulièrement citer les produits suivants :
- les gommes polydiméthylsiloxane
- les gommes polydiméthylsiloxanes/méthylvinylsiloxane,

Des produits plus particulièrement utilisables sont des mélanges tels que :
On peut aussi citer, par exemple, à titre non limitatif, les mélanges suivants :
1) les mélanges formés à partir d'une gomme polydiméthylsiloxane hydroxylé en bout de chaîne (DIMETHICONOL selon la nomenclature CTFA), et d'un polydiméthylsiloxane cyclique (CYCLOMETHICONE selon la nomenclature CTFA), tels que le produit "Q2 1401 " vendu par la Société DOW CORNING;
2) les mélanges formés à partir d'une gomme polydiméthylsiloxane avec une silicone cyclique, tel que le produit "SF 1214 SILICONE FLUID" de GENERAL ELECTRIC, qui est une gomme SE 30 de PM 500 000 solubilisée dans la "SF 1202 SILICONE FLUID" (décaméthylcyclopentasiloxane)
3) les mélanges de deux PDMS de viscosité différente, notamment d'une gomme PDMS et d'une huile PDMS, tels que les produits "SF 1236" et "CF 1241" de la Société GENERAL ELECTRIC;

Les résines d'organopolysiloxanes utilisables en tant qu'additif sont des systèmes siloxaniques réticulés renfermant les unités :
R₂SiO_{2/2}, R₃SiO_{1/2}, RSiO_{3/2} et SiO_{4/2} dans lesquelles R représente un groupement hydrocarboné possédant 1 à 16 atomes de carbone ou un groupement phényle. Parmi ces produits, ceux particulièrement préférés sont ceux dans lesquels R désigne un radical alkyle inférieur en C₁-C₄, plus particulièrement méthyle, ou un radical phényle.

On peut citer parmi ces résines le produit commercialisé sous la dénomination "DOW CORNING 593" ou ceux commercialisés sous les dénominations "SILICONE FLUID SS 4230 et SS 4267" par la société GENERAL ELECTRIC et qui sont des silicones de structure diméthyl/triméthyl siloxane.

On peut également citer les résines du type triméthylsiloxysilicate commercialisées notamment sous les dénominations X22-4914, X21-5034 et X21-5037 par la société SHIN-ETSU.

Selon l'invention, toutes les silicones peuvent être utilisées tel quel ou sous forme de solutions, d'émulsions, de nanoémulsions ou de microémulsions.

Les silicones particulièrement préférés conformément à l'invention sont :
- les silicones non volatiles choisies dans la famille des polyalkylsiloxanes à groupements terminaux triméthylsilyle telles que les huiles ayant une viscosité comprise entre 0,2 et 2,5 m²/s à 25° C telles que les huiles de la séries DC200 de DOW CORNING en particulier celle de viscosité 60 000 cSt, des séries SILBIONE 70047 et 47 et plus particulièrement l'huile 70 047 V 500 000 commercialisées par la société RHODIA CHIMIE, les polyalkylsiloxanes à groupements terminaux diméthylsilanol tels que les diméthiconol ou les polyalkylarylsiloxanes tels que l'huile SILBIONE 70641 V 200 commercialisée par la société RHODIA CHIMIE ;

Selon l'invention, les silicones additionnelles ou les autres agents bénéfiques additionnels peuvent représenter de 0,001 % à 20 % en poids, de préférence de 0,01 % à 10% en poids et plus particulièrement de 0,1 à 5% en poids par rapport au poids total de la composition finale.

Les compositions de l'invention comprennent en outre avantageusement au moins un agent tensioactif qui est généralement présent en une quantité comprise entre 0,01% et 50% en poids environ, de préférence entre 0,1% et 40% et encore plus préférentiellement entre 0,5% et 30%, par rapport au poids total de la composition.

Cet agent tensioactif peut être choisi parmi les agents tensioactifs anioniques, amphotères, non-ioniques, cationiques ou leurs mélanges.

Les tensioactifs convenant à la mise en oeuvre de la présente invention sont notamment les suivants :

### (i) Tensioactif(s) anionique(s) :

Ainsi, à titre d'exemple de tensioactifs anioniques utilisables, seuls ou mélanges, dans le cadre de la présente invention, on peut citer notamment (liste non limitative) les sels (en particulier sels de métaux alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates ; les acyliséthionates et les N-acyltaurates, le radical alkyle ou acyle de tous ces différents composés comportant de préférence de 8 à 24 atomes de carbone, et le radical aryle désignant de préférence un groupement phényle ou benzyle. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D galactoside uroniques et leurs sels ainsi que les acides alkyl (C₆-C₂₄) éther carboxyliques polyoxyalkylénés, les acides alkyl(C₆-C₂₄)aryl éther carboxyliques polyoxyalkylénés, les acides alkyl(C₆-C₂₄) amido éther carboxyliques polyoxyalkylénés et leurs sels, en particulier ceux comportant de 2 à 50 groupements oxyde d'éthylène, et leurs mélanges.

Parmi les tensioactifs anioniques, on préfère utiliser selon l'invention les sels d'alkylsulfates et d'alkyléthersufates et leurs mélanges.

### (ii) Tensioactif(s) non ionique(s) :

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178). Ainsi, ils peuvent être notamment choisis parmi (liste non limitative) les alcools, les alpha-diols polyéthoxylés, polypropoxylés ou polyglycérolés, ayant une chaîne grasse comportant par exemple 8 à 18 atomes de carbone, les alkylphénols polyéthoxylés, polypropoxylés ou polyglycérolés, ayant une chaîne grasse comportant par exemple 8 à 18 atomes de carbone ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthylène, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides et les esters d'acides gras du sorbitan oxyéthylénés ayant de 2 à 20 moles d'oxyde d'éthylène constituent des tensioactifs non-ioniques rentrant particulièrement bien dans le cadre de la présente invention.

Dans une variante préférée de l'invention, les compositions comprennent du laurate de sorbitan oxyéthyléné à 4 moles d'oxyde d'éthylène (TWEEN 21 de UNIQEMA).

### (iii) Tensioactif(s) amphotère(s):

Les agents tensioactifs amphotères peuvent être notamment (liste non limitative) des dérivés d'amines secondaires ou tertiaires aliphatiques, dans lesquels le radical aliphatique est une chaîne linéaire ou ramifiée comportant 8 à 22 atomes de carbone et comprenant au moins un groupe anionique hydrosolubilisant (par exemple carboxylate, sulfonate, sulfate, phosphate ou phosphonate) ; on peut citer encore les alkyl (C₈-C₂₀) bétaïnes, les sulfobétaïnes, les alkyl (C₈-C₂₀) amidoalkyl (C₁-C₆) bétaïnes ou les alkyl (C₈-C₂₀) amidoalkyl (C₁-C₆) sulfobétaïnes.
Parmi les alkyl (C₈-C₂₀) amidoalkyl (C₁-C₆) bétaïnes on peut citer la cocoamidopropylbétaïne vendue notamment par GOLDSCHMIDT sous le nom de TEGOBETAINE F50.

Parmi les dérivés d'amines, on peut citer les produits commercialisés sous les dénominations MIRANOL, tels que décrits dans les brevets US-2 528 378 et US-2 781 354 et de structures :

R₂ -CONHCH₂CH₂ -N(R₃)(R₄)(CH₂COO-) (2)

dans laquelle : R₂ désigne un radical alkyle dérivé d'un acide R₂-COOH présent dans l'huile de coprah hydrolysée, un radical heptyle, nonyle ou undécyle, R₃ désigne un groupement bêta-hydroxyéthyle et R₄ un groupement carboxyméthyle ; et

R₅-CONHCH₂CH₂-N(B)(C) (3)

dans laquelle :
B représente -CH₂CH₂OX', C représente -(CH₂)_{z} -Y', avec z = 1 ou 2,
X' désigne le groupement -CH₂CH₂-COOH ou un atome d'hydrogène
Y' désigne -COOH ou le radical -CH₂ - CHOH - SO3H
R₅ désigne un radical alkyle d'un acide R₉ -COOH présent dans l'huile de coprah ou dans l'huile de lin hydrolysée, un radical alkyle, notamment en C₇, C₉, C₁₁ ou C₁₃, un radical alkyle en C₁₇ et sa forme iso, un radical C₁₇ insaturé.

Ces composés sont classés dans le dictionnaire CTFA, 5ème édition, 1993, sous les dénominations Disodium Cocoamphodiacetate, Disodium Lauroamphodiacetate, Disodium Caprylamphodiacetate, Disodium Capryloamphodiacetate, Disodium Cocoamphodipropionate, Disodium Lauroamphodipropionate, Disodium Caprylamphodipropionate, Disodium Capryloamphodipropionate, Lauroamphodipropionic acid, Cocoamphodipropionic acid.
A titre d'exemple on peut citer le cocoamphodiacetate commercialisé sous la dénomination commerciale MIRANOL C2M concentré par la société RHODIA CHIMIE.

### (iv) Les tensioactifs cationiques peuvent être choisis parmi :

A) les sels d'ammonium quaternaires de la formule générale (XVI) suivante : 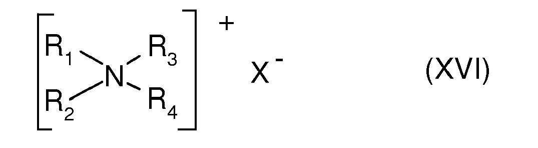 dans laquelle X est un anion choisi dans le groupe des halogénures (chlorure, bromure ou iodure) ou alkyl(C₂-C₆)sulfates plus particulièrement méthylsulfate, des phosphates, des alkyl-ou-alkylarylsulfonates, des anions dérivés d'acide organique tel que l'acétate ou le lactate.
   , et
   a) les radicaux R1 à R3, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide,
      R4 désigne un radical alkyle, linéaire ou ramifié, comportant de 16 à 30 atomes de carbone.
      De préférence le tensioactif cationique est un sel (par exemple chlorure) de béhényl triméthyl ammonium.
   b) les radicaux R1 et R2, qui peuvent être identiques ou différents, représentent un radical aliphatique, linéaire ou ramifié, comportant de 1 à 4 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, alkylamide et hydroxyalkyle, comportant environ de 1 à 4 atomes de carbone;
      R3 et R4, identiques ou différents, désignent un radical alkyle, linéaire ou ramifié, comportant de 12 à 30 atomes de carbone, ledit radical comprenant au moins une fonction ester ou amide.
      R3 et R4 sont notamment choisis parmi les radicaux alkyl(C₁₂-C₂₂)amido alkyle(C₂-C₆), alkyl(C₁₂-C₂₂)acétate ;
      De préférence le tensioactif cationique est un sel (par exemple chlorure) de stéaramidopropyl diméthyl (myristylacétate) ammonium.
B) les sels d'ammonium quaternaire de l'imidazolinium, comme par exemple celui de formule (XVII) suivante : 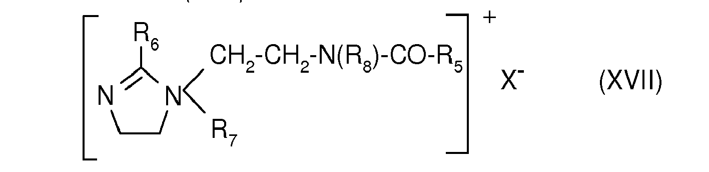 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄, R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple le Quaternium-27(CTFA 1997) ou le Quaternium-83 (CTFA 1997) commercialisés sous les dénominations "REWOQUAT" W 75, W90, W75PG, W75HPG par la société WITCO,
C) les sels de diammonium quaternaire de formule (XVIII) : 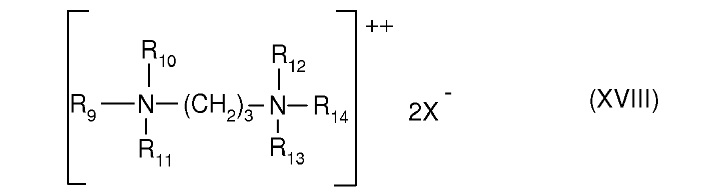 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
D) les sels d'ammonium quaternaire comprenant au moins une fonction ester de formule (XIX) suivante : 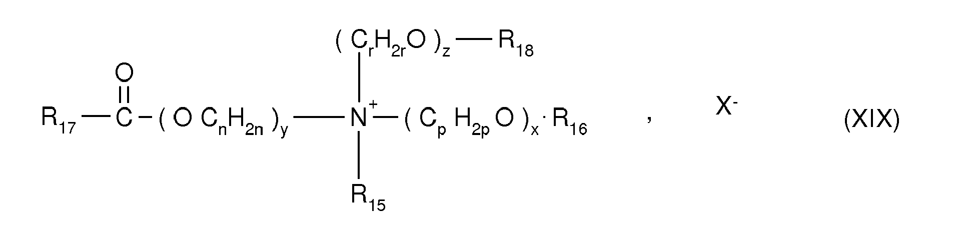 dans laquelle :
   - R₁₅ est choisi parmi les radicaux alkyles en C₁-C₆ et les radicaux hydroxyalkyles ou dihydroxyalkyles en C₁-C₆ ;
   - R₁₆ est choisi parmi :
      - le radical 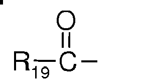
      - les radicaux R₂₀ hydrocarbonés en C₁-C₂₂ linéaires ou ramifiés, saturés ou insaturés,
      - l'atome d'hydrogène,
   - R₁₈ est choisi parmi :
      - le radical 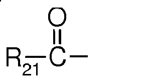
      - les radicaux R₂₂ hydrocarbonés en C₁-C₆ linéaires ou ramifiés, saturés ou insaturés,
      - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

On utilise plus particulièrement les sels d'ammonium de formule (XIX)
dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 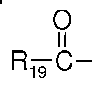
   - les radicaux méthyle, éthyle ou hydrocarbonés en C₁₄-C₂₂
   - l'atome d'hydrogène ;
- R₁₇, R₁₉ et R₂₁, identiques ou différents, sont choisis parmi les radicaux hydrocarbonés en C₇-C₂₁, linéaires ou ramifiés, saturés ou insaturés
- R₁₈ est choisi parmi :
   - le radical 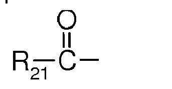
   - l'atome d'hydrogène ;

De tels composés sont par exemple commercialisés sous les dénominations DEHYQUART par la société COGNIS, STEPANQUAT par la société STEPAN, NOXAMIUM par la société CECA, REWOQUAT WE 18 par la société REWO-WITCO.

Parmi les sels d'ammonium quaternaire on préfère le chlorure de béhényltriméthylammonium, ou encore, le chlorure de stéaramidopropyldiméthyl (myristyl acétate) ammonium commercialisé sous la dénomination «CERAPHYL 70» par la société VAN DYK, le Quaternium-27 ou le Quaternium-83 commercialisés par la société WITCO.

Dans les compositions conformes à l'invention, on peut utiliser des mélanges d'agents tensioactifs et en particulier des mélanges d'agents tensioactifs anioniques, des mélanges d'agents tensioactifs anioniques et d'agents tensioactifs amphotères, cationiques ou non ioniques, des mélanges d'agents tensioactifs cationiques avec des agents tensioactifs non ioniques ou amphotères. Un mélange particulièrement préféré est un mélange comprenant au moins un agent tensioactif anionique et un ou plusieurs agent tensioactif amphotère.

La composition de l'invention peut également comprendre un ou plusieurs additif choisi parmi les épaississants, les parfums, les agents nacrants, les conservateurs, les filtres solaires siliconés ou non, les polymères anioniques ou non ioniques non siliconés, les protéines non cationiques, les hydrolysats de protéines non cationiques, l'acide méthyl-18 eicosanoique, les hydroxyacides, les vitamines, les provitamines telles que le panthénol, et tout autre additif classiquement utilisé dans le domaine cosmétique qui n'affecte pas les propriétés des compositions selon l'invention.

Les compositions conformes à l'invention peuvent également comprendre jusqu'à 5 % d'agents nacrants ou opacifiants bien connus dans l'état de la technique tels que par exemple les palmitates de sodium ou de magnésium, les stéarates et hydroxystéarates de sodium ou de magnésium, les dérivés acylés à chaîne grasse tels que les monostéarates ou distéarates d'éthylène glycol ou de polyéthylèneglycol, les éthers à chaînes grasses tels que par exemple le distéaryléther ou le 1-(hexadécyloxy)-2-octadécanol, les alcools gras en particulier les alcools stéarylique, cétylique, béhénylique et leurs mélanges.

Ces additifs sont éventuellement présents dans la composition selon l'invention dans des proportions pouvant aller de 0,001 à 20% en poids par rapport au poids total de la composition. La quantité précise de chaque additif est déterminée facilement par l'homme du métier selon sa nature et sa fonction.

Le milieu physiologiquement et notamment cosmétiquement acceptable peut être constitué uniquement par de l'eau ou par un mélange d'eau et d'un solvant cosmétiquement acceptable tel qu'un alcool inférieur en C₁-C₄, comme l'éthanol, l'isopropanol, le tertiobutanol, le n-butanol; les alkylèneglycols comme le propylèneglycol, les éthers de glycols.
De préférence, la composition comprend de 50 à 95 % en poids d'eau par rapport au poids total de la composition et plus particulièrement de 60 à 90% en poids.

Les compositions selon l'invention présentent un pH final généralement compris entre 3 et 10. De préférence, ce pH est compris entre 4 et 8, de préférence de 4 à 6. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3, ou encore par ajout d'un acide minéral ou organique, de préférence un acide carboxylique tel que par exemple l'acide citrique.

Les compositions conformes à l'invention peuvent être plus particulièrement utilisées pour le lavage ou le traitement des matières kératiniques telles que les cheveux, la peau, les cils, les sourcils, les ongles, les lèvres, le cuir chevelu et plus particulièrement les cheveux.

Les compositions selon l'invention peuvent être des compositions détergentes telles que des shampooings, des gels-douche et des bains moussants. Dans ce mode de réalisation de l'invention, les compositions comprennent au moins une base lavante, généralement aqueuse.

Le ou les tensioactifs constituant la base lavante peuvent être indifféremment choisis, seuls ou en mélanges, au sein des tensioactifs anioniques, amphotères, non ioniques et cationiques tels que définis ci-dessus. La base lavante comprend un ou plusieurs tensioactifs détergents.
Dans les compositions conformes à l'invention, on utilise de préférence au moins un ou plusieurs tensioactifs anioniques ou des mélanges d'au moins un ou plusieurs tensioactifs anioniques et d'au moins un ou plusieurs tensioactifs amphotères ou d'au moins un ou plusieurs tensioactifs non ioniques.
Un mélange particulièrement préféré est un mélange comprenant un ou plusieurs agents tensioactifs anioniques et un ou plusieurs agents tensioactifs amphotères.

On utilise de préférence un agent tensioactif anionique choisi parmi les alkyl(C₁₂-C₁₄) sulfates de sodium, de triéthanolamine ou d'ammonium, les alkyl (C₁₂-C₁₄)éthersulfates de sodium, de triéthanolamine ou d'ammonium oxyéthylénés à 2,2 moles d'oxyde d'éthylène, le cocoyl iséthionate de sodium et l'alphaoléfine(C₁₄-C₁₆) sulfonate de sodium et leurs mélange avec :
- soit un agent tensioactif amphotère tel que les dérivés d'amine dénommés disodiumcocoamphodipropionate ou sodiumcocoamphopropionate commercialisés notamment par la société RHODIA CHIMIE sous la dénomination commerciale "MIRANOL C2M CONC" en solution aqueuse à 38 % de matière active ou sous la dénomination MIRANOL C32;
- soit un agent tensioactif amphotère de type zwittérionique tel que les alkylbétaïnes ou les alkylamidobétaïnes et en particulier la cocobétaïne commercialisée sous la dénomination "DEHYTON AB 30" en solution aqueuse à 32 % de MA par la société COGNIS ou la cocoamidopropylbétaïne vendue notamment par GOLDSCHMIDT sous le nom de TEGOBETAINE F50.

La quantité et la qualité de la base lavante sont celles suffisantes pour conférer à la composition finale un pouvoir moussant et/ou détergent satisfaisant.

Ces compositions détergentes sont de préférence moussante et le pouvoir moussant des compositions selon l'invention, caractérisé par une hauteur de mousse, est généralement supérieur à 75 mm ; de préférence, supérieure à 100 mm mesurée selon la méthode ROSS-MILES (NF T 73-404 /ISO696) modifiée.
Les modifications de la méthode sont les suivantes :
La mesure se fait à la température de 22°C avec de l'eau osmosée. La concentration de la solution est de 2g/l. La hauteur de la chute est de 1m. La quantité de composition qui chute est de 200 ml. Ces 200 ml de composition tombe dans une éprouvette ayant un diamètre de 50 mm et comprenant 50 ml de la composition à tester. La mesure est faite 5 minutes après l'arrêt de l'écoulement de la composition.

Ainsi, selon l'invention, les tensioactifs détergents peuvent représenter de 3 % à 50 % en poids, de préférence de 6 % à 35 % en poids, et encore plus préférentiellement de 8 % à 25 % en poids, du poids total de la composition finale.

L'invention a encore pour objet un procédé cosmétique de traitement des matières kératiniques telles que la peau ou les cheveux, caractérisé en ce qu'il consiste à appliquer sur les matières kératiniques une composition cosmétique telle que définie précédemment, puis à effectuer éventuellement un rinçage à l'eau après un éventuel temps de pause.

Ainsi, ce procédé selon l'invention permet le maintien de la coiffure, le traitement, le soin ou le lavage ou le démaquillage de la peau, des cheveux ou de toute autre matière kératinique.

Les compositions de l'invention peuvent également se présenter sous forme d'après-shampooing à rincer ou non, de compositions pour permanente, défrisage, coloration ou décoloration, ou encore sous forme de compositions à rincer, à appliquer avant ou après une coloration, une décoloration, une permanente ou un défrisage ou encore entre les deux étapes d'une permanente ou d'un défrisage.

Lorsque la composition se présente sous la forme d'un après-shampooing éventuellement à rincer, elle comprend avantageusement un ou plusieurs tensioactifs cationiques, leurs concentrations généralement comprises entre 0,1 et 10% en poids et de préférence de 0,5 à 5% en poids par rapport au poids total de la composition.

Les compositions de l'invention peuvent encore se présenter sous la forme de compositions lavantes pour la peau, et en particulier sous la forme de solutions ou de gels pour le bain ou la douche ou de produits démaquillants.

Les compositions selon l'invention peuvent également se présenter sous forme de lotions aqueuses ou hydroalcooliques pour le soin de la peau et/ou des cheveux.

Les compositions cosmétiques selon l'invention peuvent se présenter sous forme de gel, de lait, de crème, d'émulsion, de lotion épaissie ou de mousse et être utilisées pour la peau, les ongles, les cils, les lèvres et plus particulièrement les cheveux.

Les compositions peuvent être conditionnées sous diverses formes notamment dans des vaporisateurs, des flacons pompe ou dans des récipients aérosols afin d'assurer une application de la composition sous forme vaporisée ou sous forme de mousse. De telles formes de conditionnement sont indiquées, par exemple, lorsqu'on souhaite obtenir un spray, une laque ou une mousse pour le traitement des matières kératiniques en particulier les cheveux.

Dans tout ce qui suit ou ce qui précède, les pourcentages exprimés sont en poids.

L'invention va être maintenant plus complètement illustrée à l'aide des exemples suivants qui ne sauraient être considérés comme la limitant aux modes de réalisation décrits.
Dans les exemples, MA signifie matière active.

### EXEMPLE 1

On a réalisé un après-shampooing conforme à l'invention de composition suivante :

| | |
|---|---|
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC de NOVEON) | 0,2 gMA |
| Homopolymère de chlorure de di-méthyl di-allyl ammonium en solution dans l'eau à 40 % (MERQUAT 100 de NALCO) | 0,96 gMA |
| Quaternium-80 en solution à 50% MA dans le propylène glycol (ABIL QUAT 3272 de GOLDSCHMIDT) | 0,375 gMA |
| Mono-laurate de sorbitan oxyéthyléné (20 OE) (TWEEN 20 de UNIQEMA) | 1,6 g |
| Chlorure de cétyl triméthyl ammonium en solution aqueuse à 25 % de matière active (ARQUAD 16-25 LO de AKZO NOBEL) | 0,5 gMA |
| Conservateurs | qs |
| Parfum | 0,2 g |
| Acide lactique qs | pH 4 |
| Eau desionisée qsp | 100 g |

Cette composition stable de texture agréable a été appliquée sur des cheveux très sensibilisés. Elle s'élimine aisément lors du rinçage. Les propriétés cosmétiques des cheveux traités (démêlage, lissage, souplesse) sont excellentes et homogènes des racines aux pointes des cheveux.

### EXEMPLE 2

On a réalisé un après-shampooing conforme à l'invention de composition suivante :

| | |
|---|---|
| Copolymère d'esters d'acide acrylique ou méthacrylique, de méthacrylate de C1-4 dialkylamino C1-6 alkyl, de PEG/PPG-30/5 allyl ether, de méthacrylate d'alkyl éther de PEG 20-25 C10-30, de méthacrylate d'hydroxy C2-6 alkyl réticulé avec de l'éthylène glycol diméthacrylate en émulsion à 20% dans l'eau (CARBOPOL AQUA CC de NOVEON) | 0,2 gMA |
| Homopolymère de chlorure de di-méthyl di-allyl ammonium en solution dans l'eau à 40 % (MERQUAT 100 de NALCO) | 0,96 gMA |
| Poly diméthylsiloxane à groupements aminoéthyl iminopropyle en microémulsion à 17 % dans (WACKER-BELSIL AMD LOG 1 de WACKER) | 0,5 gMA |
| Mono-laurate de sorbitan oxyéthyléné (20 OE) (TWEEN 20 de UNIQEMA) | 1,6 g |
| Chlorure de behényl triméthyl ammonium à 72.9% en solution eau/alcool isopropylique (GENAMIN KDMP de CLARIANT) | 0,7 gMA |
| Conservateurs | qs |
| Parfum | 0,2 g |
| Acide lactique qs | pH 4 |
| Eau desionisée qsp | 100 g |

Cette composition stable de texture agréable a été appliquée sur des cheveux très sensibilisés. Elle s'élimine aisément lors du rinçage. Les propriétés cosmétiques des cheveux traités (démêlage, lissage, souplesse) sont excellentes et homogènes des racines aux pointes des cheveux.

## Revendications

**1.** Composition cosmétique, **caractérisée par le fait qu'**elle comprend, dans un milieu cosmétiquement acceptable :
(i)- un ou plusieurs polymères cationiques qui sont produits par polymérisation d'un mélange de monomères comprenant :
a) un ou plusieurs monomères vinyliques substitués par un ou plusieurs groupes amino,
b) un ou plusieurs monomères vinyliques non ioniques hydrophobes, choisis parmi ceux de formules (I) et (II) :
CH₂=C(X)Z (I)
,
CH₂=CH-OC(O)R (II)
;
dans lesquelles formules (I) et (II) :
X représente H ou un groupe méthyle ;
Z est choisi parmi les groupes -C(O)OR¹, -C(O)NH₂, -C(O)NHR¹, - C(O)N(R¹)₂, -C₆H₅, -C₆H₄R¹, -C₆H₄OR¹, -C₆H₄Cl, -CN, -NHC(O)CH₃, - NHC(O)H, N-(2-pyrrolidonyle), N-caprolactamyle, -C(O)NHC(CH₃)₃, - C(O)NHCH₂CH₂-NH-CH₂CH₂-urée, -SiR₃, -C(O)O(CH₂)ₓSiR₃, - C(O)NH(CH₂)ₓSiR₃ et -(CH₂)ₓSiR₃ ;
x représente un nombre entier allant de 1 à 6 ;
chaque R représente indépendamment un groupe alkyle en C₁-C₃₀ ;
chaque R¹ représente indépendamment un groupe alkyle en C₁-C₃₀ , un groupe alkyle en C₂-C₃₀ hydroxylé, ou un groupe alkyle en C₁-C₃₀ halogéné et
c) un ou plusieurs monomères vinyliques associatifs,
e) un ou plusieurs monomères vinyliques non ioniques hydroxylés,
(ii)- une ou plusieurs silicones aminées, et
(iii)- un ou plusieurs polymères cationiques différents des polymères cationiques (i) et des silicones aminées (ii) et présentant une densité de charge cationique supérieure ou égale à 4 meq/g.

**2.** Composition selon la revendication 1, **caractérisé en ce que** le ou les monomères vinyliques substitués par un ou plusieurs groupes amino sont choisis parmi :
- les mono-(C₁-C₄)alkylamino(C₁-C₈)alkyl(méth)acrylates,
- les di-(C₁-C₄)alkylamino(C₁-C₈)alkyl(méth)acrylates, de préférence les di-(C₁-C₄)alkylamino(C₁-C₆)alkyl(méth)acrylates,
- les mono-(C₁-C₄)alkylamino(C₁-C₈)alkyl(méth)acrylamides,
- les di-(C₁-C₄)alkylamino(C₁-C₈)alkyl(méth)acrylamides,
- les (méth)acrylamides hétérocycliques comprenant un atome d'azote,
- les (méth)acrylates hétérocycliques comprenant un atome d'azote, et leurs mélanges.

**3.** Composition selon la revendication 2 **caractérisé en ce que** le ou les monomères vinyliques substitués par un ou plusieurs groupes amino sont choisis parmi :
- les (méth)acrylates de mono- ou di-(alkyl en C₁-C₄)amino(alkyle en C₁-C₄), tels que le (méth)acrylate de 2-(N,N-diméthylamino)éthyle, le (méth)acrylate de 3-(N,N-diméthylamino)propyle, le (méth)acrylate de 4-(N,N-diméthylamino)butyle, le (méth)acrylate de (N,N-diméthylamino)-t-butyle, le (méth)acrylate de 2-(N,N-diéthylamino)éthyle, le (méth)acrylate de 3-(N,N-diéthylamino)propyle, le (méth)acrylate de 4-(N,N-diéthylamino)butyle, le (méth)acrylate de 2-(N,N-dipropylamino)éthyle, le (méth)acrylate de 3-(N,N-dipropylamino)propyle et le (méth)acrylate de 4-(N,N-dipropylamino)butyle ;
- les mono- ou di-(alkyl en C₁-C₄)amino(alkyl en C₁-C₄)-(méth)acrylamides tels que le N'-(2-N,N-diméthylamino)éthyl (méth)acrylamide et le N'-(3-N,N-diméthylamino)propyl acrylamide ;
- les (méth)acrylamides ou (méth)acrylates à groupement hétérocyclique comprenant un atome d'azote, tels que le N-(2-pyridyl)acrylamide, le N-(2-imidazolyl)méthacrylamide, le méthacrylate de 2-(4-morpholinyl)éthyle, le acrylate de 2-(4-morpholinyl)éthyle, le N-(4-morpholinyl)méthacrylamide et le N-(4-morpholinyl)acrylamide ; et
- les hétérocycles azotés à groupement(s) vinyle(s) tels que la 2-vinylpyridine et la 4-vinylpyridine.

**4.** Composition selon l'une quelconque des revendications 1 à 3, **caractérisée en ce que** le ou les monomères vinyliques substitués par un ou plusieurs groupes amino représentent généralement de 10 à 70 % en poids, de préférence de 20 à 60 % en poids, par rapport au poids total du mélange de monomères.

**5.** Composition selon l'une quelconque des revendications précédentes, **caractérisée en ce que** le ou les monomères vinyliques non ioniques hydrophobes sont choisis parmi les (méth)acrylates d'alkyle en C₁-C₃₀, les (alkyl en C₁-C₃₀)(méth)acrylamides, le styrène, les styrènes substitués, les esters de vinyle, les nitriles insaturés, et les silanes insaturés.

**6.** Composition selon l'une quelconque des revendications précédentes, **caractérisée en ce que** le ou les monomères vinyliques non ioniques hydrophobes représentent généralement de 20 à 80% en poids, de préférence de 20 à 70% en poids, par rapport au poids total du mélange de monomères.

**7.** Composition selon l'une quelconque des revendications précédentes, **caractérisée en ce que** le ou les monomères vinyliques associatifs sont choisis parmi les composés de formule (III) : 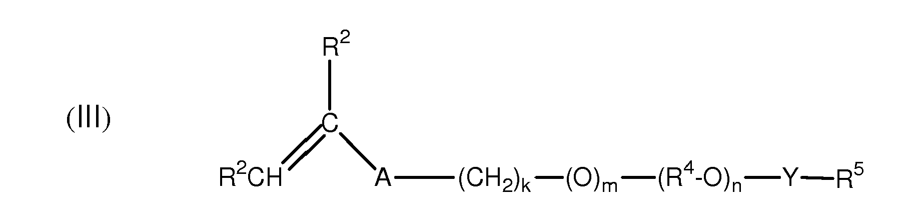 dans laquelle
chaque R² est indépendamment H, un groupe méthyle, un groupe -C(O)OH, ou un groupe -C(O)OR³ ;
R³ est un alkyle en C₁-C₃₀ ;
A est un groupe -CH₂C(O)O-, -C(O)O-, -O-, CH₂O, -NHC(O)NH-, -C(O)NH-, -Ar-(CE₂)_{z}-NHC(O)O-, -Ar-(CE₂)_{z} -NHC(O)NH-, ou -CH₂CH₂-NHC(O)-;
Ar est un groupe aryle divalent ;
E est H ou un groupe méthyle ;
z est égal à 0 ou 1 ;
k est un entier variant de 0 à 30 ;
m est égal à 0 ou 1, à la condition que lorsque k = 0, m = 0, et lorsque k varie de 1 à 30, m est égal à 1 ;
(R⁴-O)ₙ est un polyoxyalkylène, qui est un homopolymère, un copolymère aléatoire, ou un copolymère à blocs, avec des unités oxyalkylène en C₂-C₄,
R⁴ est C₂H₄, C₃H₆, C₄H₈, ou leurs mélanges,
n est un entier variant de 5 à 250,
Y est -R⁴O-, -R⁴NH-, -C(O)-, -C(O)NH-, R⁴NHC(O)NH-, ou - C(O)NHC(O)-;
R⁵ est un alkyle substitué ou non choisi parmi les alkyles linéaires en C₈-C₄₀, les alkyles ramifiés en C₈-C₄₀, les alicycliques en C₈-C₄₀, les phényles substitués par un groupe alkyle en C₂-C₄₀, les alkyles en C₂-C₄₀ substitués par un groupe aryle, les esters complexes en C₈-C₈₀,
le groupe alkyle R⁵ comprenant éventuellement un ou plusieurs substituants choisis parmi les groupes hydroxyle, alcoxyle et halogèno.

**8.** Composition selon la revendication 7, **caractérisée en ce que** le ou les monomères vinyliques associatifs sont choisis parmi les (méth)acrylates polyéthoxylés de cétyle, les (méth)acrylates polyéthoxylés de cétéaryle, les (méth)acrylates polyéthoxylés de stéaryle, les (méth)acrylates polyéthoxylés d'arachidyle, les (méth)acrylates polyéthoxylés de béhényle, les (méth)acrylates polyéthoxylés de lauryle, les (méth)acrylates polyéthoxylés de cérotyle, les (méth)acrylates polyéthoxylés de montanyle, les (méth)acrylates polyéthoxylés de mélissyle, les (méth)acrylates polyéthoxylés de laccéryle, les (méth)acrylates polyéthoxylés de 2,4,6-tri(1'-phényléthyl)phényle, les (méth)acrylates polyéthoxylés de l'huile de ricin hydrogénée, les (méth)acrylates polyéthoxylés de canola, les (méth)acrylates polyéthoxylés du cholestérol et leurs mélanges, où la portion polyéthoxylée du monomère comprend de 5 à 100, de préférence de 10 à 80, et mieux de 15 à 60 unités oxydes d'éthylène.

**9.** Composition selon l'une quelconque des revendications précédentes, **caractérisée en ce que** le ou les monomères vinyliques associatifs représentent de 0,001 à 25 % en poids, de préférence de 0,01 à 15 % en poids du mélange de monomères.

**10.** Composition selon l'une quelconque des revendications précédentes, **caractérisée en ce que** le mélange de monomères comprend un ou plusieurs monomères tensioactifs vinyliques semi-hydrophobes d) choisis parmi les composés de formules (IV) ou (V) : 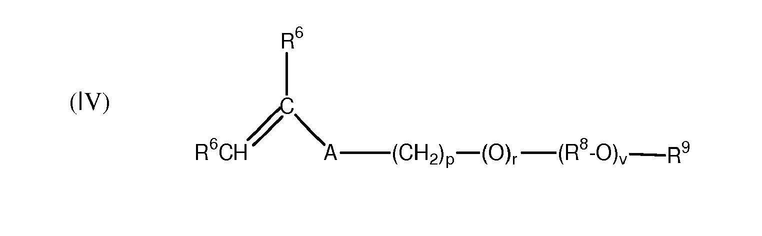
D-A-(CH₂)ₚ-(O)ᵣ-(R⁸-O)ᵥ-R⁹ (V)
où, dans chaque formule (IV) ou (V),
chaque R⁶ représente indépendamment H, un alkyle en C₁-C₃₀, -C(O)OH, ou C(O)OR⁷ ;
R⁷ est un alkyle en C₁-C₃₀ ;
A est un groupe -CH₂C(O)O-, -C(O)O-, -O-, -CH₂O, -NHC(O)NH-, -C(O)NH-, -Ar-(CE₂)_{z}-NHC(O)O-, -Ar-(CE₂)_{z} -NHC(O)NH-, ou -CH₂CH₂NHC(O)-
Ar est un groupe aryle divalent ;
E est H ou un groupe méthyle ;
z est égal à 0 ou 1 ;
p est un entier variant de 0 à 30 ;
r est égal à 0 ou 1, à la condition que lorsque p est égal à 0, r est égal à 0, et lorsque p varie de 1 à 30, r est égal 1,
(R₈-O)ᵥ est un polyoxyalkylène qui est homopolymère, un copolymère aléatoire, ou un copolymère à blocs avec des unités oxyalkylène en C₂-C₄, où R⁸ est C₂H₄, C₃H₆, C₄H₈, ou leurs mélanges, et v est un entier variant de 5 à 250 ;
R⁹ est H ou un alkyle en C₁-C₄ ;
D est un alcényle en C₈-C₃₀ ou un alcényle en C₈-C₃₀ substitué par un groupe carboxy.

**11.** Composition selon la revendication 10 **caractérisée en ce que** le mélange de monomères comprend un ou plusieurs monomères tensioactifs vinyliques semi-hydrophobes ayant l'une des formules suivantes :
CH₂=CH-O(CH₂)ₐO(C₃H₆O)_{b}(C₂H₄O)_{c}H ou CH₂=CHCH₂O(C₃H₆O)_{d}(C₂H₄O)ₑH;
où
a est égal à 2, 3, ou 4 ;
b est un entier variant de 1 à 10 ;
c est un entier variant de 5 à 50 ;
d est un entier variant de 1 à 10 ; et
e est un entier variant de 5 à 50.

**12.** Composition selon l'une quelconque des revendications précédentes, **caractérisée en ce que** le ou les monomères tensioactifs vinyliques semi-hydrophobes représentent de 0 à 25 % en poids, de préférence de 0,1 à 10 % en poids du mélange de monomères.

**13.** Composition selon l'une quelconque des revendications précédentes, **caractérisée en ce que** le ou les monomères vinyliques non ioniques hydroxylés d) sont choisis parmi les (méth)acrylates d'hydroxyalkyle en C₁-C₆, les (hydroxyalkyl en C₁-C₄)(méth)acrylamides, et leurs mélanges.

**14.** Composition selon la revendication 13, **caractérisée en ce que** le monomère vinylique non ionique hydroxylé est le méthacrylate de 2-hydroxyéthyle.

**15.** Composition selon l'une quelconque des revendications précédentes, **caractérisée en ce que** le ou les monomères vinyliques non ioniques hydroxylés représentent de préférence de 0,01 à 10 % en poids du mélange de monomères.

**16.** Composition selon la revendication précédente **caractérisée en ce que** le mélange de monomères comprend, par rapport au poids total du mélange de monomères :
a) de 20 à 60 % en poids du ou des monomères vinyliques substitués par un ou plusieurs groupes amino,
b) de 20 à 70 % en poids du ou des monomères vinyliques non ioniques hydrophobes,
c) de 0,01 à 15 % en poids du ou des monomères vinyliques associatifs,
d) de 0,1 à 10 % en poids du ou des monomères tensioactifs vinyliques semi-hydrophobes,
e) de 0,01 à 10 % en poids du ou des monomères vinyliques non ioniques hydroxylés,
f) de 0,001 à 5 % en poids du ou des monomères réticulants, et
g) de 0,001 à 10% en poids du ou des agents de transfert de chaîne,
h) de 0 à 2 % en poids du ou des stabilisateurs polymériques.

**17.** Composition selon l'une quelconque des revendications précédentes **caractérisée en ce que** ledit mélange de monomères comprend :
- un méthacrylate de di(alkyl en C₁-C₄)amino(alkyle en C₁-C₆),
- un ou plusieurs esters d'alkyle en C₁-C₃₀ et de l'acide (méth)acrylique,
- un méthacrylate d'alkyle en C₁₀-C₃₀ polyéthoxylé avec 20 à 30 moles d'oxyde d'éthylène,
- un allyl éther de polyéthylèneglycol/polypropylèneglycol 30/5,
- un méthacrylate d'hydroxy(alkyle en C₂-C₆),
- un diméthacrylate d'éthylèneglycol.

**18.** Composition selon l'une quelconque des revendications précédentes, **caractérisée en ce que** le ou les polymères cationiques (i) sont présents à une concentration allant de 0,01 à 10% en poids par rapport au poids total de la composition, de préférence de 0,05 à 5% en poids et mieux de 0,1 à 1% en poids.

**20.** Composition selon l'une quelconque des revendications précédentes, **caractérisée en ce que** la silicone aminée (ii) est présente à une concentration comprise entre 0,001 % et 20 % en poids par rapport au poids total de la composition, de préférence entre 0,01 % et 10 % en poids.

**21.** Composition selon l'une quelconque des revendications précédentes, **caractérisée par le fait que** ledit polymère cationique ayant une densité de charge cationique supérieure à 4 meq/g est choisi parmi les homopolymères du chlorure de diallyldiméthylammonium et les copolymères du chlorure de diallyldiméthylammonium et d'acrylamide.

**22.** Composition selon l'une quelconque des revendications précédentes, **caractérisée en ce que** le polymère cationique ayant une densité de charge cationique supérieure à 4 meq/g est présent à une concentration allant de 0,001 % à 10 % en poids, de préférence de 0,005 % à 5 % en poids, et encore plus préférentiellement de 0,01 % à 3 % en poids, du poids total de la composition finale.

**23.** Utilisation d'une composition telle que définie dans l'une quelconque des revendications précédentes pour le lavage ou pour le soin des matières kératiniques.

**24.** Procédé cosmétique de traitement des matières kératiniques, telles que les cheveux, **caractérisé en ce qu'**il consiste à appliquer sur lesdites matières une composition cosmétique selon l'une quelconque des revendications 1 à 22, puis à effectuer éventuellement un rinçage à l'eau après un éventuel temps de pause.

**25.** Utilisation d'une composition telle que définie dans l'une quelconque des revendications 1 à 22 pour apporter une ou plusieurs propriétés choisies parmi la brillance, la légèreté des cheveux, la douceur, le lissage au toucher, et/ou de la souplesse des cheveux.
